# FASCICULE DE BREVET EUROPEEN

(11) **EP 3 914 154 B1**
(45) Date de publication et mention de la délivrance du brevet: **19.11.2025**
(21) Numéro de dépôt: 20706574.9
(22) Date de dépôt: 24.01.2020
(51) Int. Cl.: A61B 5/053, A61B 5/00, A61B 5/24, A61B 5/392

(54) **DISPOSITIF DE MESURE D'UNE CONGESTION DU TRACTUS DIGESTIF**
VORRICHTUNG ZUR MESSUNG EINER VERSTOPFUNG DES VERDAUUNGSTRAKTS
DEVICE FOR MEASURING A CONGESTION OF THE DIGESTIVE TRACT

(30) Priorité: 24.01.2019 FR 1900629
(43) Date de publication de la demande: 01.12.2021
(73) Titulaire: Sentinhealth, 75001 Paris (FR); Université Grenoble Alpes, 38400 Saint-Martin-d'Hères (FR)
(72) Inventeur: DOPIERALA, Cindy, 75001 Paris (FR); POIZAT, Adrien, 75001 Paris (FR); PARMENTIER, Thibault, 75001 PARIS (FR); GUMERY, Pierre-Yves, 38000 Grenoble (FR); BOUCHER, François, 75001 Paris (FR)
(74) Mandataire: Ex Materia
(86) Numéro de dépôt international: PCT/FR2020/050114
(87) Numéro de publication internationale: WO 2020/152429

(56) Documents cités:
- CN-A- 101 622 784
- US-A1- 2009 275 824
- US-A1- 2010 228 105
- US-A1- 2013 150 685
- US-A1- 2017 224 986
- PANAGIOTIS KASSANOS ET AL: "A tetrapolar bio-impedance sensing system for gastrointestinal tract monitoring", 2015 IEEE 12TH INTERNATIONAL CONFERENCE ON WEARABLE AND IMPLANTABLE BODY SENSOR NETWORKS (BSN), 1 June 2015 (2015-06-01), pages 1 - 6, XP055631088, ISBN: 978-1-4673-7201-5, DOI: 10.1109/BSN.2015.7299403

## Description

Le domaine de la présente invention est celui des dispositifs de mesure appliqués aux composantes corporelles, plus particulièrement les dispositifs aptes à mesurer la bio-impédance d'un corps humain au niveau du tractus digestif. Le terme « tissu » utilisé ci-après, désignera l'ensemble des tissus du tractus digestif concerné par la mesure d'impédance, et plus précisément les tissus du tractus gastro-intestinal.

De façon connue, des pathologies telles qu'une insuffisance cardiaque peuvent provoquer chez l'Homme une accumulation de liquide dans les poumons. Cette accumulation aqueuse, autrement appelée œdème, empêche la correcte respiration : se logeant dans les tissus pulmonaires au niveau extracellulaire, l'eau constitue un obstacle aux échanges gazeux d'oxygène et de gaz carbonique qui y ont lieu.

Une décompensation cardiaque peut ainsi conduire à un œdème cardiogénique au niveau thoracique, notamment dans le tissu pulmonaire. Une gêne respiratoire et un essoufflement font partie des symptômes, induits par une compensation des poumons augmentant leur travail respiratoire et provoquant notamment une douleur thoracique. Ces symptômes s'aggravent jusqu'à la détresse respiratoire majeure si l'accumulation de liquide n'est pas détectée en amont. L'œdème pulmonaire est considéré médicalement comme une urgence vitale qu'il est nécessaire de traiter dès les premiers signes, les traitements étant d'autant plus lourds que le diagnostic est tardif, les tissus étant moins engorgés à un stade initial.

L'œdème pulmonaire est un stade tardif de la maladie dont le traitement repose entre autres sur l'usage de médicaments diurétiques, dont l'efficacité à ce stade de la maladie est insuffisante par voie orale (même à forte dose), nécessitant hospitalisation et traitement diurétiques par voie intraveineuse. L'explication de la baisse d'efficacité de ces médicaments par voie orale est la présence d'eau (œdème) dans la paroi gastro-intestinale, empêchant leur absorption. Il est même vraisemblable, de par la gravité, que l'œdème pulmonaire soit postérieur à l'œdème de la paroi gastrique et intestinale. Ainsi la mesure de la présence d'eau au niveau du tractus digestif pourrait être un signe précoce de l'étape ultérieure qu'est la présence d'eau dans les poumons, et ce d'autant que 35% des patients insuffisants cardiaques ont une augmentation de la perméabilité digestive (Sandek A, Bauditz J, Swidsinski A, Buhner S, Weber-Eibel J, von Haehling S et al. Altered Intestinal Function in Patients With Chronic Heart Failure. JACC October 2007. Vol. 50, No. 16, 2007, 2007:1561-9*)*

Tout patient présentant un risque de pathologie cardiaque doit ainsi être vigilant à cause de la possible apparition d'un œdème pulmonaire. Les patients à risque sont généralement suivis par des examens médicaux réguliers, type auscultation par le praticien, radiographie pulmonaire, dosage sanguin et/ou électrocardiogramme afin d'identifier des troubles cardiaques. Préventivement, le patient est contraint à surveiller étroitement son hygiène de vie et à traiter sa pathologie cardiaque pour éviter des complications sous-jacentes à l'œdème pulmonaire.

Un suivi médical reste cependant contraignant pour le patient, puisqu'il est dépendant du milieu médical et du praticien pour effectuer une évaluation de son état pulmonaire et cardiaque. Un autre inconvénient réside dans la régularité du suivi, le patient devant fréquemment se soumettre au milieu médical pour prévenir des complications aggravées. Par ailleurs, le suivi ne peut raisonnablement être effectué de façon pluri journalière au long terme, pour tous les patients, notamment ceux gardant une bonne autonomie.

Le document US 2017/224986 A1 concerne un dispositif, un système et une méthode pour diagnostiquer et traiter les troubles gastriques.

Le document US 2013/150685 A1 concerne un système et une méthode de surveillance continue d'une anastomose gastro-intestinale.

L'invention est définie par les revendications.

Le but de la présente divulgation est donc de résoudre les inconvénients décrits ci-dessus en concevant un dispositif de bio-impédancemétrie utilisé pour détecter avec robustesse des changements de teneur en eau, ou de congestion, dans le tissu gastrique afin de détecter le développement d'un œdème viscéral, d'utilisation simple et répétée pour le suivi indirect et précoce d'un patient présentant un risque d'œdème pulmonaire.

La divulgation concerne un dispositif de mesure de congestion du tractus digestif d'un utilisateur, le dispositif de mesure comprenant au moins un boîtier, un générateur de courant et un moyen de mesure de différence de potentiel logés chacun dans ledit boîtier, et un jeu d'électrodes comportant au moins deux électrodes raccordées électriquement, indépendamment l'une de l'autre, au générateur de courant et/ou au moyen de mesure de différence de potentiel aux bornes des électrodes, chaque électrode du jeu d'électrodes étant configurée pour émettre un courant électrique et/ou permettre la mesure d'une différence de potentiel électrique. Le jeu d'électrodes est configuré pour générer au moins une boucle de circulation du courant électrique circulant au moins au travers d'un tissu gastrique ou intestinal de l'utilisateur et pour permettre la mesure d'une différence de potentiel électrique relatif au tissu gastrique ou intestinal, et le dispositif de mesure comprend en outre un module de calcul configuré pour recevoir la différence de potentiel électrique mesurée et pour calculer une valeur de bio-impédance du tractus digestif en fonction de cette mesure relative au tissu gastrique ou intestinal. Un tel dispositif de mesure de congestion du tractus digestif permet ainsi notamment de mesurer une bio-impédance des tissus du tractus digestif.

Une mesure automatisée d'une variation du volume de liquide dans le tissu gastrique ou intestinal permettrait d'évaluer l'apparition d'un œdème en relative autonomie. La mesure de la bio-impédance est une mesure renseignant notamment sur une composition en liquide du corps, comme la présence d'un œdème. Des dispositifs connus de mesure de la bio-impédance ne permettent cependant pas d'évaluer de façon spécifique et robuste la présence d'un œdème thoracique. De plus, aucun dispositif connu ne permet d'évaluer la présence d'un œdème du tractus digestif.

Selon une caractéristique de la divulgation, le dispositif de mesure comporte des moyens distincts permettant une mesure mécanique d'une caractéristique structurelle de la paroi gastrique.

Le dispositif de mesure selon la divulgation met ainsi en œuvre, dans le cadre d'une approche multimodale, des moyens de mesure d'une impédance électrique du tissu gastrique et des moyens distincts permettant une mesure mécanique d'une caractéristique structurelle ou morphologique de la paroi gastrique.

Le dispositif de mesure peut être alors défini comme multimodale en ce qu'il permet la mesure et la prise en compte de signaux de bio-impédance électrique d'une part et de signaux mécaniques d'autre part. L'analyse croisée de ces données, et par exemple lorsque chacune de ces données dépasse une valeur seuil, permet de déterminer de manière fiable si le tissu gastrique est congestionné, c'est-à-dire qu'il est rempli de liquide, une telle détermination permettant par la suite à un professionnel médical de diagnostiquer une éventuelle insuffisance cardiaque.

Selon une caractéristique de la divulgation, les moyens permettant une mesure mécanique d'une caractéristique structurelle ou morphologique de la paroi gastrique consistent en un accéléromètre. Un tel capteur est notamment configuré pour détecter les vibrations et pour quantifier, par au moins une grandeur définie, ces vibrations au niveau de la paroi gastrique.

Il est particulièrement avantageux de lier l'évolution des caractéristiques des ondes sismocardiographiques en réponse à un choc cardiaque, par analyse du signal accélérométrique recueilli par l'accéléromètre, avec les différents niveaux d'impédance électrique du tissu gastrique. L'analyse des caractéristiques du signal accélérométrique, par exemple l'énergie ou la largeur de bande de fréquence des vibrations de la paroi gastrique, permet de mettre en évidence des indications de modifications structurelles et morphologiques de la paroi gastrique. La combinaison de cette information avec l'analyse des niveaux d'impédance permet de déduire que le changement structurel et morphologique détecté est dû à une présence accrue de liquide dans la paroi gastrique.

Une base d'apprentissage utilisant des méthodologies d'apprentissage automatique peut être utilisée pour détecter des variations caractéristiques liées à l'apparition d'un œdème viscéral.

Selon une caractéristique de la divulgation, les moyens permettant une mesure mécanique d'une caractéristique structurelle ou morphologique de la paroi gastrique, notamment l'accéléromètre, sont intégrés dans le boîtier du dispositif de mesure précédemment évoqué.

Avantageusement, les moyens permettant une mesure mécanique d'une caractéristique structurelle ou morphologique de la paroi gastrique, notamment l'accéléromètre, sont ainsi agencés à proximité des électrodes permettant la mesure d'un signal accélérométrique dans une zone du tissu gastrique correspondant à la zone dans laquelle la mesure de bio impédance a été effectué. Dans ce contexte, il est également plus facile de recueillir les données mesurées dans un même module de calcul, configuré pour traiter les données mesurées et notamment les comparer à des valeurs seuils pour détecter le cas échéant le dépassement d'un niveau d'alerte susceptible de déclencher une démarche médical ultérieure de diagnostic.

Cette proximité mécanique, du fait de l'intégration des capteurs et électrodes dans un même boîtier, permet en outre d'optimiser la synchronisation des mesures. Tel que cela sera évoqué ci-après, les mesures de bio-impédance et de signal mécanique, ici accélérométrique, sont réalisées simultanément pour avoir une base de comparaison fiable et la synchronisation du lancement de ces deux mesures est facilité par un trajet court des instructions de commande entre le module de calcul par exemple et les capteurs et électrodes.

Le boîtier et le jeu d'électrodes sont configurés pour être internalisés, c'est à dire implantés dans l'utilisateur. Ils sont configurés pour être, au moins le boîtier et le cas échéant tout ou partie des électrodes, en contact direct avec les tissus de l'utilisateur, et plus particulièrement en contact prolongé avec les tissus de l'utilisateur. Dans ce contexte, le boîtier et le jeu d'électrodes peuvent être réalisés dans des matériaux inertes pour le corps. Par exemple, le boîtier et le jeu d'électrodes peuvent être réalisés dans des matériaux biocompatibles ou comprendre un revêtement biocompatible. Quels que soient les matériaux employés, le jeu d'électrodes conserve ses propriétés conductrices émettrices et/ou réceptrices.

Le module de calcul est configuré pour traiter les données de mesure de différence de potentiel électrique et notamment pour traiter l'évolution de ces données mesurées par rapport à des seuils prédéfinis, et pour en déduire une valeur de bio-impédance du tractus gastrique, dont on peut tirer une information relative à l'état de l'utilisateur. Il comporte au moins des moyens de communication lui permettant de recevoir la différence de potentiel électrique mesurée aux bornes des électrodes alimentées par le générateur de courant. L'appareil mis en œuvre pour mesurer cette différence de potentiel électrique, à savoir un voltmètre, et le module de calcul sont reliés en filaire ou communiquent par ondes, selon le mode de réalisation. Le module de calcul peut être embarqué dans le boîtier ou bien être déporté à distance du boîtier, sans sortir du contexte de l'invention, dès lors que le boîtier intègre au moins un générateur de courant et un dispositif de mesure aptes à être reliés électriquement, indépendamment les uns des autres, aux électrodes, déportées hors du boîtier ou non.

Qu'il soit embarqué ou déporté par rapport au boîtier, le module de calcul peut être, dans un mode de réalisation, configuré pour être internalisé, c'est-à-dire implanté dans l'utilisateur. Il comporte alors des moyens de communication lui permettant de transmettre la valeur de bio-impédance du tractus digestif calculée, ou l'information relative à un état de l'utilisateur. Par exemple, le module de calcul comprend des émetteurs. Dans un autre mode de réalisation, le module de calcul est externalisé.

Le jeu d'électrodes d'au moins deux électrodes, que celles-ci soient intégrées au boîtier ou déportées hors du boîtier, est raccordé au générateur de courant et au moyen de mesure de différence de potentiel, chaque électrode du jeu étant indépendamment l'une de l'autre reliée électriquement à ces composants, en étant disposée au moins au contact du tissu gastrique ou intestinal par lequel on souhaite faire passer la boucle de circulation de courant. Par exemple, chacune des électrodes du jeu d'électrodes comprend au moins une surface d'interface destinée à être au contact du tissu gastrique ou intestinal. Avantageusement, le jeu d'électrodes est configuré pour être disposé dans le tissu gastrique ou intestinal.

Le courant électrique amené à circuler à travers le corps de l'utilisateur via les électrodes est un courant électrique de faible intensité. Par faible intensité on entend notamment un courant d'intensité inférieure au milliampère. Plus particulièrement, à titre d'exemple, le courant électrique peut être un courant alternatif, sinusoïdal, d'une intensité de l'ordre de 50 à 700 microampères pour une fréquence de l'ordre de 5kHz à 1MHz, la mesure pouvant être multi fréquentielle.

La mesure de différence de potentiel électrique est effectuée aux bornes des électrodes, pour un courant d'intensité constante, d'une valeur d'impédance, selon la loi d'Ohm. La différence de potentiel électrique résultant du passage du courant d'une électrode à l'autre, dans un sens ou dans l'autre étant entendu que les électrodes peuvent être à la fois injectrice et réceptrice, est modifiée en fonction de la résistance des tissus biologiques rencontrés dans la zone gastrique ou intestinale traversée. La mesure aux bornes des électrodes disposées sur le trajet du courant permet de quantifier la variation de ce potentiel électrique et donc la variation de bio-impédance pour un courant d'intensité constante.

La mesure de grandeurs électriques de courant circulant qui est réalisée par le dispositif selon l'invention répond à plusieurs impératifs. Il convient de réaliser une mesure locale afin de pouvoir réaliser une mesure suffisamment spécifique de la bio-impédance dans le tissu gastrique ou intestinal, et que cette mesure locale soit ciblée dans la sous-muqueuse et/ou le cas échéant dans la muqueuse, afin d'éviter un éventuel shunt électrique dû au contenu de la lumière gastrique ou intestinal. Il convient également de réaliser cette mesure sur une distance suffisamment grande pour avoir une mesure globale fiable ne tenant pas compte de l'hétérogénéité des tissus présents dans ces différentes parois. Et dans ce contexte, différentes caractéristiques de l'invention telles qu'elles vont être décrites plus en détail par la suite visent à proposer un dispositif dans lequel une pluralité d'électrodes sont disposées en redondance les unes derrière les autres, reliées électriquement indépendamment l'une de l'autre au générateur de courant, rendant possible des mesures de proche en proche sans que les boucles de circulation de courant ne s'étendent de façon préjudiciable dans la cavité de l'estomac.

Selon la divulgation, une mesure accélérométrique est réalisée au temps t. Les valeurs recueillies par l'accéléromètre sont envoyées au module de calcul pour extraire la signature accélérométrique de la paroi gastrique en réponse au choc cardiaque. Tel que cela a été évoqué, le module de calcul peut être invasif et agencé dans le boîtier du dispositif de mesure ou bien être externalisé sans que l'on sorte pour autant du contexte de la divulgation, dès lors que les moyens mécaniques et électriques sont implantés dans le même boîtier. Simultanément, on mesure une valeur d'impédance électrique du tissu gastrique. La signature accélérométrique de la paroi gastrique et des valeurs d'impédance électrique au cours du temps est analysée notamment en les comparant à des valeurs seuils. Dans le cas où l'évolution de ces deux mesures, électriques et mécaniques, est cohérente, et que les valeurs recueillies dépassent chacune une valeur seuil correspondante préalablement définie, une information est envoyée sur une base de données et/ou un moyen de traitement de données externe, étant entendu que cette information est à considérer comme un possible œdème viscéral à diagnostiquer par la suite.

Les inventeurs ont en effet considéré que, dans le cas d'une rétention hydrique de la paroi gastrique, d'une part l'impédance électrique du tissu va varier, la résistance diminue du fait de la présence d'eau qui facilite le transfert du courant d'une électrode du boîtier à l'autre, et d'autre part la paroi gastrique subit également des modifications structurelles du fait de la présence d'eau, dont un épaississement de la paroi, qui entraîne une modification de la signature accélérométrique, les ondes provoquées par le choc cardiaque n'étant alors pas transmises de la même manière.

La prise en compte des signaux électriques et mécaniques acquis simultanément grâce au dispositif de mesure selon la divulgation permet ainsi de donner une information fiable sur l'existence d'une rétention hydrique.

Selon un aspect de la divulgation le dispositif de mesure est dimensionné pour être implanté au niveau du tissu du tractus gastro-intestinal par voie endo-luminale ou par chirurgie abdominale. Pour être implanté au niveau du tissu du tractus gastro-intestinal par voie endo-luminale, le dispositif de mesure est dimensionné pour être inséré via la cavité buccale de l'utilisateur et son tube digestif. Par exemple, le dispositif de mesure est dimensionné pour être inséré dans ou porté par une sonde endo-luminale, telle un endoscope. L'implantation par voie endo-luminale peut être couplée à un acte chirurgical destiné à finaliser l'implantation du dispositif de mesure. Alternativement, le dispositif de mesure est destiné à être implanté par un acte chirurgical opéré directement au niveau de l'abdomen.

Selon un aspect de la divulgation, le jeu d'électrodes comporte deux électrodes parmi lesquelles une première électrode disposée à une première extrémité longitudinale du boîtier et une deuxième électrode disposée à une deuxième extrémité longitudinale opposée du boîtier. La disposition du jeu d'électrodes aux extrémités du boîtier permet de mettre en œuvre une mesure de différence de potentiel électrique en local, c'est-à-dire sur la distance de quelques centimètres que présente le boîtier. La boucle de circulation passe ainsi de la première électrode à la deuxième électrode en passant par le tissu gastrique ou intestinal à proximité du boîtier, entre la première extrémité longitudinale et la deuxième extrémité longitudinale du boîtier. On comprend que cette mesure, simple car n'impliquant pas d'électrodes ou de fils de connexion électrique en dehors du boîtier, implique une mesure très locale, qui peut varier fortement selon la zone d'implantation du boîtier en fonction de l'hétérogénéité des tissus.

La première électrode comprend une première surface d'interface configurée pour être au contact d'une première zone du tractus gastro-intestinal. La deuxième électrode comprend une deuxième surface d'interface configurée pour être au contact d'une deuxième zone du tractus gastro-intestinal, distincte de la première. Par exemple, pour que le contact première électrode/première zone gastrique ou intestinale soit assuré tout comme le contact deuxième électrode/deuxième zone gastrique ou intestinale, la première surface d'interface affleure la première extrémité longitudinale du boîtier et la deuxième surface d'interface affleure la deuxième extrémité longitudinale du boîtier.

La surface d'interface de chaque électrode est étendue selon une certaine superficie. Par exemple, sans que cela soit limitatif de l'invention, la surface d'interface de chaque électrode peut être comprise entre 20 et 40 mm². Dans un autre exemple, la surface d'interface de chaque électrode peut correspondre à 15% de la surface totale du boîtier, +/- 10%.

La première électrode est, dans un exemple de réalisation, étendue sur toute la première extrémité longitudinale du boîtier. Dans ce même exemple de réalisation, la deuxième électrode peut aussi être étendue sur toute la deuxième extrémité longitudinale du boîtier. Dans un exemple alternatif de réalisation, la première électrode occupe une partie de la première extrémité longitudinale du boîtier. Dans ce même exemple de réalisation, la deuxième électrode peut aussi occuper une partie de la deuxième extrémité longitudinale du boîtier. Par exemple, la première électrode et la deuxième électrode ont une forme de vignette posée en surface du boîtier respectivement au niveau de la première extrémité longitudinale et de la deuxième extrémité longitudinale. Avantageusement, la première surface d'interface et la deuxième surface d'interface sont orientées dans le même sens, la première électrode et la deuxième électrode sont alors disposées d'un même côté sur le boîtier, les normales à chaque surface d'interface étant parallèles.

Selon un aspect de la divulgation, le jeu d'électrodes comprend une pluralité d'électrodes parmi lesquelles au moins une électrode est déportée à l'extérieur du boîtier, et dans lequel un support d'électrodes déportées externe au boîtier est configuré pour relier une des bornes du générateur de courant et/ou du moyen de mesure de différence de potentiel à ladite électrode déportée, le support d'électrodes déportées comprenant une gaine périphérique isolante s'étendant entre le boîtier et ladite électrode déportée

Selon un aspect de la divulgation, le jeu d'électrodes comprend une pluralité d'électrodes, disposées géométriquement les unes après les autres en s'éloignant du boîtier, le cas échéant en étant sensiblement alignées.

Dans cette configuration, la distance la plus grande possible entre deux électrodes du jeu d'électrodes est augmentée par rapport à une configuration où les électrodes sont logées dans le boîtier. Ceci permet d'avoir une bonne couverture du champ de mesure de bio-impédance sur le tissu gastrique ou intestinal, alors que le boîtier reste relativement petit de sorte à minimiser l'impact invasif du boîtier et du support d'électrodes déportées.

Le support d'électrodes déportées est situé hors du boîtier. Il est isolé électriquement par la gaine périphérique qui l'entoure, et relie au moins une électrode déportée au générateur de courant et/ou au moyen de mesure de différence de potentiel électrique. Afin que les distances inter-électrode soient conservées lorsque le dispositif de mesure selon l'invention est installé, le support d'électrodes déportées peut présenter une certaine rigidité. Par exemple, c'est la gaine périphérique isolante qui permet d'assurer cette rigidité. Avantageusement, la gaine périphérique isolante peut être semi-rigide de sorte à ne pas gêner l'utilisateur.

Dans ce qui précède et ce qui va suivre, le terme d'électrodes est utilisé pour désigner de manière globale une électrode seule ou un ensemble d'électrodes disposées côte à côte dans une zone définie. Le dispositif de mesure selon la divulgation peut être mis en œuvre aussi bien avec une mesure de bio-impédance à deux électrodes, qui se comprend alors par une circulation de courant entre une électrode émettrice/réceptrice et une autre électrode émettrice/réceptrice à distance l'une de l'autre, ou bien avec une mesure de bio-impédance à quatre électrodes, qui se comprend alors par une circulation de courant entre un ensemble d'électrodes émettrices et un ensemble d'électrodes réceptrices, ces ensembles étant à distance l'un de l'autre.

Dans ce dernier cas notamment, un ensemble d'électrodes peut être configuré pour injecter du courant et former une boucle de circulation et pour d'autre part recevoir du courant d'une autre boucle de circulation lorsque des mesures locales distinctes sont mises en œuvre simultanément ou alternativement.

Selon un aspect de la divulgation, toutes les électrodes du jeu d'électrodes sont déportées à l'extérieur du boîtier, la gaine périphérique isolante logeant, de manière isolée électriquement les uns par rapport aux autres, une pluralité de cordons conducteurs d'électricité configurés pour relier les électrodes déportées au générateur de courant et/ou au moyen de mesure de différence de potentiel, indépendamment les unes des autres. On pourra identifier parmi les électrodes, disposées géométriquement les unes après les autres dans le sens de l'éloignement du boîtier, des électrodes extrêmes avec une première électrode extrême la plus proche du boîtier et une deuxième électrode extrême la plus éloignée du boîtier, et une ou plusieurs électrodes intermédiaires. Il convient de noter que le terme intermédiaire se réfère alors à la position géométrique de l'électrode et non à ses caractéristiques techniques. La ou les électrodes intermédiaires sont déportées par rapport au boîtier.

Selon un aspect de la divulgation, le jeu d'électrodes forme une matrice monodimensionnelle s'étendant depuis le boîtier jusqu'à l'électrode la plus éloignée du boîtier, en passant par chacune des électrodes intermédiaires. La pluralité d'électrodes du jeu d'électrodes ainsi formé permet de réaliser plusieurs mesures locales de différence de potentiel électrique. Chaque mesure de potentiel électrique en local correspond à une boucle de circulation locale, correspondant à la boucle de circulation la plus courte réalisée par des électrodes du jeu d'électrodes, de proche en proche. Cette redondance dans le positionnement des électrodes, reliées électriquement au générateur de courant indépendamment les unes des autres, permet de réaliser des mesures de proche en proche et d'étendre la distance sur laquelle la mesure est effectuée tout en assurant que les boucles de circulation ne pénètrent pas ou peu dans la lumière gastro-intestinale, à l'intérieur de laquelle la conductivité du contenu luminal gastrique ou intestinal pourrait fausser l'analyse de la mesure de différence de potentiel électrique aux bornes des électrodes.

On comprend qu'une mesure en plusieurs points successifs permet d'étendre la portée de la mesure et donc de proposer une mesure globale fiable, non dépendante de l'hétérogénéité des tissus. A titre d'exemple, on pourra prévoir quatre électrodes intermédiaires disposées entre le boîtier et l'électrode extrême la plus éloignée du boîtier, sans que cela soit limitatif. On comprendra que l'on pourra proposer, sans sortir du contexte de l'invention, un nombre différent d'électrodes dès lors que la fermeture d'une boucle de circulation de courant est générée de proche en proche, tel que cela a été évoqué ci-dessus.

Selon un aspect de la divulgation, une distance égale peut séparer chaque électrode de son électrode immédiatement voisine. En d'autres termes, une même distance inter-électrode sépare deux à deux les électrodes du jeu d'électrodes.

Selon un aspect de la divulgation, chaque électrode déportée hors du boîtier comprend une surface d'interface avec le tissu gastrique ou intestinal. Le dispositif peut être implanté de sorte que les surfaces d'interface de ces électrodes soient orientées dans une même direction et un même sens, la direction et le sens de la surface d'interface étant définis par rapport à une normale au plan dans lequel la surface d'interface s'étend. Le dispositif peut être implanté de sorte que les électrodes aient leur surface d'interface en contact avec le tissu gastrique ou intestinal, de sorte à s'assurer que la boucle de circulation de courant passe le plus possible au travers du tissu gastrique ou intestinal. Avantageusement, l'intégralité de la boucle de circulation passe au travers du tissu gastrique ou intestinal. Ainsi, la mesure de potentiel électrique effectuée, révélatrice de l'état de bio-impédance du tractus digestif, est spécifique du tissu et non des environnements adjacents comme la cavité délimitée par la paroi gastrique ou intestinale.

Selon un aspect de la divulgation, le jeu d'électrodes comporte au moins une électrode intermédiaire s'étendant à l'extérieur du boîtier entre le boîtier et ladite électrode déportée formant l'électrode la plus éloignée du boîtier, au moins une électrode intermédiaire étant disposée à l'extrémité d'une branche conductrice de courant et isolée du tissu du tractus digestif par une gaine isolante, ladite branche étant déployable par rapport au cordon muni de la gaine périphérique isolante.

Le courant électrique circule dans le cordon conducteur d'électricité du support d'électrodes déportées et dans la branche conductrice de courant pour alimenter, indépendamment l'une de l'autre, les électrodes du dispositif et permettre de former chaque boucle de circulation à considérer pour déterminer la valeur de bio-impédance gastrique. La branche conductrice déployable permet d'éloigner l'électrode qu'elle porte du support d'électrodes déportées. Lorsque le support d'électrodes déportées est implanté, éloigner les électrodes permet de s'affranchir de la dégénérescence fibreuse du tissu qui survient autour du support d'électrodes déportées suite à l'implantation du boîtier du dispositif auquel est raccordé le cordon. La mesure de différence de potentiel électrique réalisée est alors plus robuste. En position de mise en œuvre, la branche conductrice de courant s'étend sensiblement perpendiculairement par rapport au support d'électrodes déportées. Afin d'être maintenue dans cette position, la branche conductrice de courant est avantageusement rigide.

Avant la mise en place du dispositif de mesure selon la divulgation, la branche conductrice de courant peut être en position repliée ou rétractée. Dans la position repliée ou rétractée, la branche conductrice s'étend le long du support d'électrodes déportées. Une fois que le dispositif de mesure est en position de mise en œuvre, la branche conductrice est en position déployée de sorte à éloigner l'électrode qu'elle porte du support d'électrodes déportées. Le caractère déployable de la branche conductrice de courant est compatible avec un déploiement au sein du tissu gastrique ou intestinal. Ainsi, le déploiement doit se faire malgré la résistance que peuvent rencontrer la branche conductrice de courant et l'électrode qu'elle porte. Pour être déployable, la branche conductrice de courant est par exemple associée à un mécanisme de déploiement. Par exemple, le mécanisme de déploiement est un mécanisme de déploiement à cran ou à ressort.

La branche conductrice de courant est biocompatible, tout comme le mécanisme de déploiement qu'elle contient.

Selon une caractéristique de l'invention, le jeu d'électrodes associé au boîtier comporte une pluralité d'électrodes reliées électriquement au générateur de courant et/ou au moyen de mesure de différence de potentiel, que les électrodes soient logées dans le boîtier ou déportées hors du boîtier, et le dispositif de mesure comporte un jeu de commutateurs disposés sur les liaisons électriques indépendantes les unes des autres de chaque électrodes avec le générateur de courant et/ou le moyen de mesure de différence de potentiel, le module de calcul étant configuré pour piloter l'ouverture et la fermeture de chaque commutateur pour déterminer quelles électrodes du jeu sont mises en œuvre pour émettre et mesurer le courant traversant le tissu gastrique ou intestinal. La mise en œuvre de ces commutateurs pilotés permet de sélectionner quelle paire d'électrodes est choisie pour former la boucle de circulation de courant à considérer. Et il est alors possible de programmer une séquence d'ouverture et de fermeture des commutateurs pour réaliser une succession de mesures locales entre deux électrodes voisines, le cas échéant immédiatement voisines, afin de moyenner ensuite ces mesures locales pour définir une mesure globale.

Tel que précédemment décrit, le dispositif de mesure peut être configuré pour permettre une mesure à deux électrodes, ou une mesure à quatre électrodes. Dans la mesure à deux électrodes, chaque électrode est reliée à la fois au générateur de courant et au dispositif de mesure de la différence de potentiel, de manière à fonctionner en injection et en réception de courant traversant le tissu gastrique ou intestinal. Et dans la mesure à quatre électrodes, une paire d'électrodes est reliée aux bornes du générateur de courant et une autre paire d'électrodes est reliée aux bornes du moyen de mesure de différence de potentiel, chaque électrode pouvant fonctionner respectivement en émission ou réception de courant.

Selon un aspect de la divulgation, le dispositif de mesure comprend au moins un dispositif de fixation au tissu gastrique ou intestinal. Le dispositif de fixation correspond à tout élément permettant de solidariser le dispositif de mesure avec le tissu gastrique ou intestinal. Ainsi immobilisé, le dispositif de mesure de bio-impédance du tractus digestif permet d'obtenir des mesures de différence de potentiel fiables, car sans approximation sur la position réelle des électrodes les unes par rapport aux autres. Le dispositif de fixation est par exemple muni d'une pince apte à fixer le dispositif de mesure au tissu gastrique ou intestinal.

Selon un aspect de la divulgation, au moins une extrémité du boîtier comprend un dispositif de fixation au tissu gastrique ou intestinal. En d'autres termes, le dispositif de mesure peut comprendre un dispositif de fixation au tissu gastrique ou intestinal disposé à la première extrémité longitudinale et/ou un dispositif de fixation à la deuxième extrémité longitudinale. Avantageusement, le dispositif de fixation au tissu gastrique ou intestinal, est disposé sur chaque extrémité du boîtier à l'opposé de la surface d'interface des électrodes. Ainsi, la circulation du courant n'est pas perturbée.

Selon un aspect de la divulgation, le support d'électrodes déportées comprend tout ou partie du dispositif de fixation au tissu gastrique ou intestinal. Le dispositif de fixation peut ainsi être présent au niveau du support d'électrodes déportées. L'orientation et la position des électrodes portées par le support d'électrodes déportées, à savoir la troisième électrode et potentiellement la série d'électrodes intermédiaires, sont assurées, garantissant la reproductibilité des mesures de potentiel électrique. Le support d'électrodes déportées peut associer un dispositif de fixation à chaque électrode.

Selon un aspect de la divulgation, au moins la branche conductrice de courant comprend un dispositif de fixation au tissu gastrique ou intestinal. Lorsque la branche conductrice de courant est apte à être déployée, le dispositif de fixation est positionné de sorte à ne pas entraver ce déploiement. Le dispositif de fixation, ou dispositif d'ancrage, est fixé au tissu gastrique ou intestinal, après le déploiement de la branche conductrice de courant.

Ainsi, au moins une extrémité du boîtier et/ou le support d'électrodes déportées et/ou la branche conductrice de courant comprend le dispositif de fixation au tissu gastrique ou intestinal.

La divulgation concerne également un procédé de mesure de congestion du tractus digestif, mettant en œuvre le dispositif de mesure tel que précédemment décrit, le procédé comprenant une étape de mesure de différence de potentiel électrique et une étape de calcul, l'étape de calcul étant mise en œuvre par un module de calcul apte à recevoir au moins une mesure de différence de potentiel électrique et à calculer une valeur de bio-impédance du tractus digestif à partir de la mesure de différence de potentiel électrique.

L'étape de mesure de différence de potentiel électrique inclut l'injection de courant par les électrodes aptes à injecter du courant la réception par les électrodes réceptrices du courant ayant parcouru la boucle de circulation au travers du tissu gastrique ou intestinal.

L'étape de calcul est mise en œuvre par le module de calcul. Le module de calcul intègre la mesure de différence potentiel électrique ou les mesures de différence potentiel électrique locales, selon le nombre d'électrodes mises en œuvre dans le jeu d'électrodes. Le module de calcul détermine une valeur de bio-impédance du tractus digestif grâce aux mesures de différence de potentiel électrique réalisées aux bornes des électrodes.

Selon un aspect de la divulgation, la mesure de différence de potentiel électrique correspond à la somme d'une pluralité de mesures locales de différence de potentiel électrique, chaque mesure locale de différence de potentiel électrique résultant d'un raccordement électrique au moins à un générateur de courant d'une paire d'électrodes immédiatement adjacentes du jeu d'électrodes du dispositif de mesure. Pendant l'étape de mesure, les mesures de différence de potentiel électrique locales sont collectées. Chaque mesure de différence de potentiel électrique locale correspond à la différence de potentiel électrique mesurée pour une boucle de circulation la plus courte possible, pour des électrodes de proche en proche.

D'autres caractéristiques, détails et avantages de l'invention ressortiront plus clairement à la lecture de la description donnée ci-après à titre indicatif en relation avec des dessins dans lesquels :
- [Fig.1] est une vue générale, en perspective, d'un dispositif de mesure de congestion du tractus digestif selon la divulgation dans un premier mode de réalisation,
- [Fig.2] est une vue des éléments implantés du dispositif de mesure présenté en figure 1 dans un mode de fonctionnement,
- [Fig.3] est une vue générale, en perspective, des éléments destinés à être implantés d'un dispositif de mesure selon la divulgation dans un deuxième mode de réalisation,
- [Fig.4] est une vue des éléments implantés du dispositif de mesure présenté en figure 3 dans un mode de fonctionnement,
- [Fig.5] est une vue générale, en perspective, des éléments destinés à être implantés d'un dispositif de mesure selon la divulgation dans un troisième mode de réalisation,
- [Fig.6] est une vue des éléments implantés du dispositif de mesure présenté en figure 3 dans un mode de fonctionnement,
- [Fig.7] et la [Fig.8] sont des vues en situation des éléments implantés du dispositif de mesure selon la divulgation dans un quatrième et un cinquième mode de réalisation,
- [Fig.9] est une vue générale, en perspective, des éléments destinés à être implantés d'un dispositif de mesure selon la divulgation dans un sixième mode de réalisation,
- [Fig.10] est une vue des éléments implantés du dispositif de mesure présenté en figure 9 dans un mode de fonctionnement,
- [Fig.11] et [Fig.12] décrivent un mode d'installation des éléments destinés à être implantés du dispositif de mesure présenté à la figure 9,
- [Fig.13], [Fig.14] et [Fig.15] illustrent des schémas de raccordement électrique appropriés pour le bon fonctionnement du dispositif de mesure selon la divulgation, et
- [Fig.16] et [Fig.17] illustrent respectivement plus en détails le fonctionnement d'une mesure dite à deux électrodes et à quatre électrodes.

Il faut tout d'abord noter que les figures exposent la divulgation de manière détaillée pour mettre en œuvre la divulgation, lesdites figures pouvant bien entendu servir à mieux définir la divulgation le cas échéant.

Dans la suite de la description, les dénominations longitudinales ou latérales, dessus, dessous, devant, derrière se réfèrent à l'orientation du boîtier du dispositif de mesure de congestion du tractus digestif selon la divulgation. La direction longitudinale correspond à l'axe principal du boîtier dans lequel il s'étend, alors que les orientations latérales correspondent à des droites concourantes, c'est-à-dire qui croisent la direction longitudinale, notamment perpendiculaires à l'axe longitudinal du boîtier.

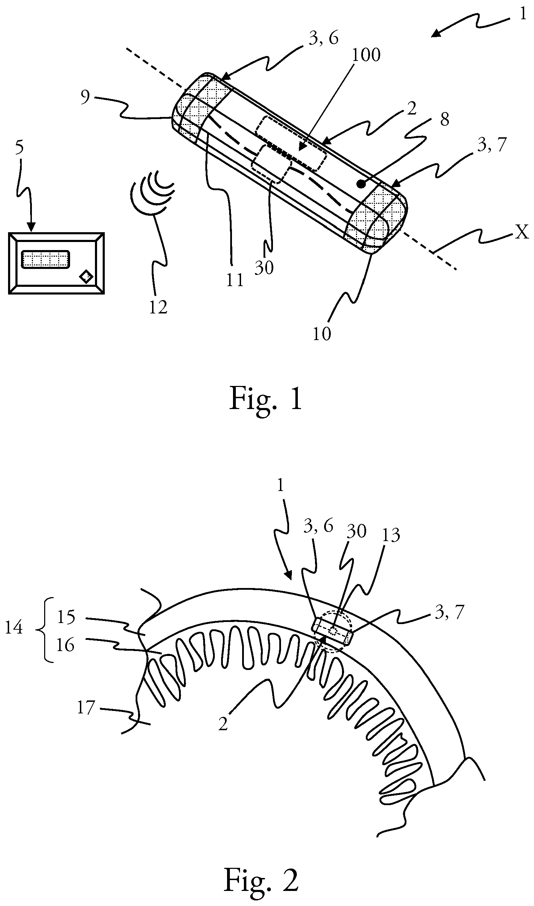

En se référant tout d'abord à la figure 1, on voit un dispositif de mesure 1 de congestion du tractus digestif d'un utilisateur. Le dispositif de mesure 1 comporte au moins un boîtier 2, un jeu 3 d'électrodes raccordées au boîtier 2 ainsi qu'un générateur de courant 30 abrité dans le boîtier et relié électriquement et indépendamment l'une de l'autre à chaque électrode du jeu 3 d'électrodes. Le dispositif de mesure 1 comprend en outre un moyen de mesure de différence de potentiel, par exemple un voltmètre, 31 représenté schématiquement tout comme le générateur de courant.

Le dispositif de mesure comporte en outre un capteur configuré pour relever un signal mécanique révélateur d'une structure, forme ou morphologie du tractus digestif dans lequel est implanté le dispositif de mesure. Selon l'exemple illustré, le capteur est un accéléromètre 100 logé dans le boîtier. L'accéléromètre 100 est disposé dans le boîtier dans une position sensiblement centrale, au voisinage du générateur de courant 30.

Le dispositif de mesure 1 comporte encore un module de calcul 5 apte à recevoir une différence de potentiel électrique mesurée entre deux électrodes du jeu 3 d'électrodes, de manière à calculer une valeur de bio-impédance du tractus digestif et des valeurs de signaux mesurés par le capteur, ici l'accéléromètre 100.

Dans un premier exemple de réalisation, illustré sur la figure 1, le jeu 3 d'électrodes comprend au moins deux électrodes 6, 7 disposées dans le volume du boîtier et qui participent, de par leur connexion électrique respective au générateur de courant 30, à la formation d'une boucle de circulation fermée lorsque le dispositif de mesure 1 est positionné dans un milieu conducteur, et plus particulièrement dans des tissus conducteurs de l'utilisateur, comme cela est décrit en figure 2.

Le boîtier 2 est une structure fermée possédant une surface externe 8. Le boîtier 2 étend sa plus grande dimension selon un axe longitudinal X. Par exemple, le boîtier 2, mesuré selon l'axe longitudinal X, mesure entre 2 et 4 cm.

Le boîtier 2 comprend une première extrémité longitudinale 9 et une deuxième extrémité longitudinale 10 opposée à la première extrémité longitudinale 9. Dans l'exemple de réalisation de la figure 1, une première électrode 6 du jeu 3 d'électrodes est disposée à la première extrémité longitudinale 9 du boîtier 2 et une deuxième électrode 7 du jeu 3 d'électrodes est disposée à la deuxième extrémité longitudinale 10 du boîtier 2. Dans cet exemple de réalisation, la première électrode 6 et la deuxième électrode 7 sont raccordées à la surface externe 8 du boîtier 2 et recouvrent respectivement la première extrémité longitudinale 9 et la deuxième extrémité longitudinale 10. Les électrodes sont représentées en grisé pour faciliter leur détection par le lecteur.

Le boîtier 2 est constitué d'un matériau isolant, de sorte à ce que le boîtier 2 en lui-même soit non conducteur d'électricité.

Chaque électrode du jeu 3 d'électrodes a une surface d'interface destinée à être au contact d'un tissu gastrique ou intestinal de l'utilisateur. Chaque électrode du jeu 3 d'électrodes est apte à fonctionner comme électrode injectrice de courant et/ou comme électrode de mesure permettant de mesurer une différence de potentiel électrique d'une électrode à l'autre alors que le courant devant circuler d'une électrode à l'autre pour fermer la boucle de circulation passe par le tissu gastrique ou intestinal. La même électrode du jeu 3 d'électrodes peut simultanément avoir ces deux fonctionnalités, émettrice et réceptrice, dans les modes de réalisation ici illustrés.

Le boîtier 2 est fait d'un matériau biocompatible. Il est de forme parallélépipédique et il comporte des bords roulés 11 de sorte à préserver les tissus qui lui sont environnants une fois implanté. Le boîtier 2 peut prendre toute forme compatible avec son utilisation. Avantageusement, le boîtier 2 est de forme oblongue pour un impact réduit sur les tissus environnants et pour en faciliter le positionnement.

Le module de calcul 5 est ici illustré externalisé par rapport au boîtier 2. Il comporte des moyens de communication de sorte à recevoir le potentiel électrique mesuré. Par exemple, le module de calcul 5 comprend un récepteur. Le jeu 3 d'électrodes est alors relié à un émetteur permettant de transmettre l'information mesurée par le moyen de mesure de différence de potentiel électrique au module de calcul 5, comme illustré par des ondes 12. L'émetteur est ici compris dans le boîtier 2, et relié au jeu 3 d'électrodes. En variante non illustré on pourrait prévoir que le module de calcul est intégré dans le boîtier.

La figure 2 montre le dispositif de mesure 1 de congestion du tractus digestif mis en œuvre au cours d'un procédé de mesure de congestion du tractus digestif, le dispositif de mesure 1 étant ici un dispositif gastrique ou intestinal implanté dans une sous-muqueuse. Cette implantation n'est pas limitative, et on comprend que le dispositif selon la divulgation peut être implanté dans tout autre tissu du tractus digestif.

Le jeu 3 d'électrodes est configuré pour générer au moins la boucle de circulation 13 du courant électrique lorsque le générateur de courant 30 est mis en œuvre. Cette boucle de circulation 13 circule alors au moins d'une électrode à une autre au travers d'un tissu du tractus gastro-intestinal 14 de l'utilisateur comme montré en figure 2. Dans cet exemple de réalisation, le boîtier 2 et le jeu 3 d'électrodes sont disposés dans un tel tissu 14. De façon particulière, le boîtier 2 et le jeu 3 d'électrodes sont intégrés dans un tissu sous-muqueux 15 du tissu 14 de l'utilisateur. Ainsi, la boucle de circulation 13 circule au moins au travers du tissu sous-muqueux 15 du tissu 14 de l'utilisateur.

Dans un exemple de réalisation, la boucle de circulation 13 traverse le tissu sous-muqueux 15 et une muqueuse 16 du tissu 14, la muqueuse 16 séparant le tissu sous-muqueux 15 d'une cavité 17 délimitée par le tissu 14 et étant inclue dans le tissu 14. Selon la position du jeu 3 d'électrodes, une partie de la boucle de circulation 13 peut être amenée à circuler hors du tissu 14, par exemple dans la cavité 17 délimitée par le tissu du tractus gastro-intestinal 14.

Il faut comprendre que le boîtier 2 et le jeu 3 d'électrodes pourraient être disposés différemment dès lors que la position du jeu 3 d'électrodes permet la formation d'une boucle de circulation 13 fermée traversant au moins le tissu du tractus gastro-intestinal 14.

Le procédé de mesure de congestion du tractus digestif, mis en œuvre par le dispositif de mesure 1, comprend au moins une étape de mesure de différence de potentiel électrique et une étape de calcul. Il comprend également une mesure mécanique d'une caractéristique structurelle ou morphologique de la paroi gastrique, et notamment une mesure d'ondes suite à un choc cardiaque par l'intermédiaire d'un accéléromètre 100.

Lors de l'étape de mesure de différence de potentiel électrique, au moins une électrode, ici la première électrode 6, du jeu 3 d'électrodes émet dans la muqueuse ou la sous-muqueuse, le courant provenant du générateur 30, ce courant étant amené à traverser le tissu du tractus gastrointestinal 14 en direction d'une autre électrode, ici la deuxième électrode 7, du jeu d'électrodes. Le courant emprunte le cheminement le plus court pour fermer la boucle de circulation 13.

Un voltmètre, formant moyen de mesure de différence de potentiel 31, est branché aux bornes du générateur de courant pour déterminer une mesure de différence de potentiel électrique d'une électrode à l'autre, cette mesure de différence de potentiel électrique étant le reflet de la facilité de circulation de courant à travers les tissus gastriques et donc le reflet de la présence de liquide dans ces tissus, tel que l'on peut en déduire un état de bio-impédance du tractus digestif.

L'information de différence de potentiel électrique reçue est transmise au module de calcul 5, ici au moyen de l'émetteur qui est internalisé dans le boîtier dans cet exemple de réalisation.

L'étape de calcul est mise en œuvre par le module de calcul 5 externalisé, ici non représenté mais visible en figure 1. Grâce à la mesure d'une différence de potentiel électrique, le module de calcul 5 calcule une valeur de bio-impédance du tractus digestif. Le résultat est alors informatif quant à l'état de bio-impédance du tractus digestif de l'utilisateur, et permet, par exemple, d'envoyer à l'utilisateur ou à un personnel médical approprié une information sur l'état de santé de l'utilisateur, et par exemple sur un possible état de décompensation cardiaque de l'utilisateur. On notera que l'utilisation de la mesure d'une bio-impédance du tractus digestif de l'utilisateur n'est pas limitée à donner une information sur l'état de décompensation cardiaque de l'utilisateur, et qu'elle peut permettre de caractériser d'autre informations relatives à l'état de santé de l'utilisateur.

La mesure mécanique, et notamment accélérométrique, prévue de manière additionnelle et simultanée à la mesure de bio-impédance, est réalisée en réponse à un choc cardiaque survenant lors de la période de mesure. L'accéléromètre est apte à quantifier l'onde provoqué par le choc cardiaque telle qu'elle est transmise à travers la paroi gastrique. Des variations dans le temps du signal mécanique ainsi acquis sont notamment dues à une modification structurelle de la paroi gastrique, qui ne transmet pas de la même manière les ondes selon sa configuration.

Les valeurs recueillies par l'accéléromètre sont envoyées au module de calcul pour extraire la signature accélérométrique de la paroi gastrique en réponse au choc cardiaque.

L'étape de calcul précédemment évoquée, mise en œuvre par le module de calcul, tient alors compte des signaux mécaniques acquis. Avantageusement, le même module de calcul est utilisé pour récupérer et traiter les données électriques et mécaniques acquises par les capteurs et électrodes.

Grâce à la mesure d'au moins une caractéristique du signal accélérométrique, le module de calcul 5 calcule une valeur représentative d'une caractéristique structurelle ou morphologique du tractus digestif, dans la même zone que celle sur laquelle a été effectuée la mesure de bio-impédance. La combinaison de ces deux valeurs, et notamment la combinaison des comparaisons des ces valeurs avec une valeur seuil qui leur est associée et stockée dans une mémoire du module de calcul, est alors informative quant à la présence de liquide dans le tractus digestif de l'utilisateur, et permet, par exemple, d'envoyer à l'utilisateur ou à un personnel médical approprié une information sur l'état de santé de l'utilisateur, et par exemple sur un possible état de décompensation cardiaque de l'utilisateur.

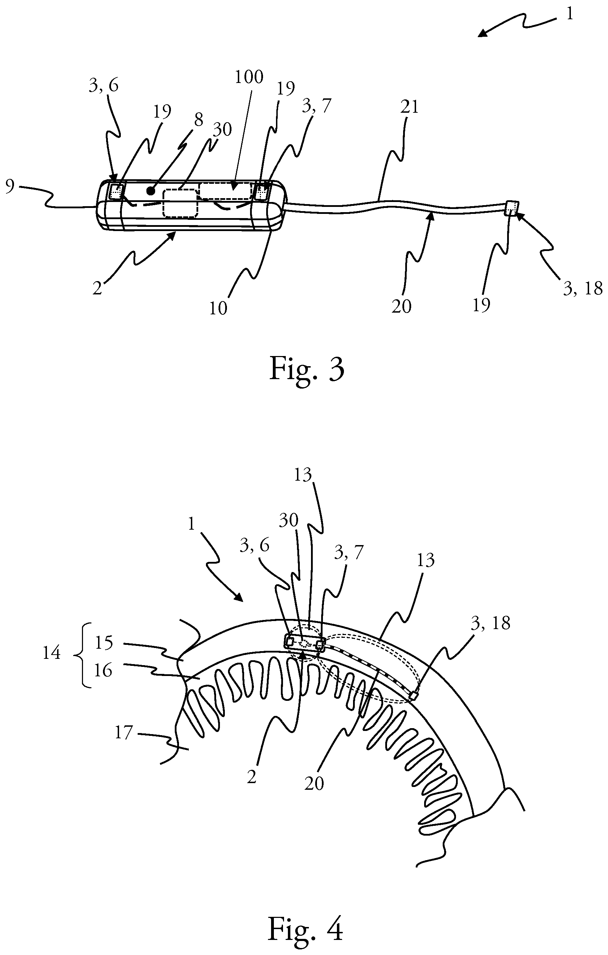

La figure 3 illustre un mode de réalisation dans lequel le dispositif de mesure 1 diffère de celui décrit en figure 1 par ce qui va suivre. Le dispositif de mesure 1 comprend, dans son jeu 3 d'électrodes, une électrode dite troisième électrode 18, déportée par rapport au boîtier 2. La première électrode, la deuxième électrode et la troisième électrode sont reliées électriquement au générateur de courant 30, indépendamment l'une de l'autre, de manière à participer à l'émission d'une boucle de réalisation de courant qui parcourt ainsi une plus grande distance globale au travers du tissu 14 entre la première électrode 6 et la troisième électrode 18. La mesure n'est pas localisée sur la dimension longitudinale du boîtier 2 mais sur une distance allant de la première électrode 6 à la troisième électrode 18.Plus particulièrement, et tel que cela est visible sur la figure 4, la distance globale entre la première électrode et la troisième électrode est parcourue par une première boucle de circulation 13 s'étendant entre la première électrode et la deuxième électrode, et par une deuxième boucle de circulation 13 s'étendant entre la deuxième électrode et la troisième électrode à distance du boîtier. On comprend que l'étude des potentiels électriques pour chacune des boucles permet de considérer de façon globale la conductivité des tissus sur une distance allant de la première électrode à la troisième électrode.

Dans cet exemple de réalisation, la première électrode 6 et la deuxième électrode 7 sont raccordées à la surface externe 8 du boîtier 2. Chaque électrode du jeu 3 d'électrodes a une surface d'interface 19, orientée dans une même direction et destinée à être au contact du tissu 14. Les surfaces d'interface 19 ont leurs normales parallèles entre elles et, de plus, les surfaces d'interface 19 ont la même orientation par rapport au tissu 14, par exemple par rapport au tissu sous-muqueux 15. Les surfaces d'interface 19 prennent la forme de pastilles ayant une forme quelconque dès lors que le positionnement relatif des surfaces d'interface 19 est respecté.

La troisième électrode 18 est ici identique à la première électrode 6 et à la deuxième électrode 7, si ce n'est son caractère déporté du boîtier 2. Un support d'électrodes déportées 20, externe au boîtier 2, s'étend depuis la deuxième extrémité longitudinale du boîtier de manière à supporter au moins la troisième électrode 18, à distance de ce boîtier. La troisième électrode 18 est reliée électriquement au générateur de courant 30, indépendamment des autres électrodes. A cet effet, le support d'électrodes déportées 20 comprend au moins un cordon conducteur d'électricité et une gaine périphérique 21 isolante s'étendant depuis le boîtier jusqu'à la troisième électrode 18.

Il convient de noter que le dispositif de mesure comporte là encore un accéléromètre 100, qui est cette fois décalé à proximité de la deuxième électrode 7, afin de prendre une position la plus centrée possible sur la distance entre les deux électrodes les plus éloignées l'une de l'autre. Conformément au premier mode de réalisation précédemment décrit, il est ici cherché à centrer la zone du tractus gastrique dans laquelle est réalisée la mesure de signaux mécaniques sur la zone de ce tractus gastrique dans laquelle est réalisée la mesure de bio-impédance.

La figure 4 montre notamment la boucle de circulation 13 générée par la deuxième électrode 7 et la troisième électrode 18. La figure illustre également la circulation de courant en direction de la troisième électrode, le long du cordon conducteur d'électricité dans le support d'électrodes déportées 20 comme signifié en pointillés. Pour les autres éléments illustrés par la figure 4, l'on pourra se reporter *mutatis mutandis* à la description faite pour la figure 2. On pourra ainsi se référer à la figure 2 pour la mise en œuvre et la compréhension de la divulgation selon le mode de réalisation décrit en figure 4.

Le courant électrique est émis depuis le générateur en trois points du jeu 3 d'électrodes, c'est à dire au niveau de chacune des électrodes, de manière isolée électriquement les unes par rapport aux autres. Tel qu'illustré, une distance inter-électrode plus grande sépare la deuxième électrode 7 et la troisième électrode 18 par rapport à la première électrode 6 et la deuxième électrode 7. La boucle de circulation 13 générée entre la deuxième électrode 7 et la troisième électrode 18 est donc plus grande que celle générée entre la première électrode 6 et la deuxième électrode 7, le courant ayant plus de distance à parcourir pour relier la deuxième électrode 7 et la troisième électrode 18. On comprend qu'une mesure de proche en proche telle qu'illustrée permet d'éviter que la boucle de circulation 13 ne s'écarte trop du jeu 3 d'électrodes et passe à travers la cavité 17. Les électrodes sont ainsi positionnées et agencées les unes par rapport aux autres de manière à pouvoir former des boucles de circulation 13 évitant la cavité 17, à l'intérieur duquel le liquide gastrique pourrait former un shunt électrique.

Un tel dispositif de mesure 1 génère deux mesures locales de différence de potentiel électrique, effectuées entre deux électrodes immédiatement adjacentes par le moyen de mesure de différence de potentiel électrique. La mesure de différence de potentiel électrique utilisée pour calculer la valeur de bio-impédance du tractus digestif correspond à la somme de ces deux mesures locales de différence de potentiel électrique, dès lors que le courant injecté localement lors des mesures de différences de potentiels est le même d'une mesure locale à l'autre. Alternativement, il peut être prévu de déterminer des valeurs de bio-impédance locales, qui sont calculées respectivement à partir des mesures locales de différence de potentiel électrique. Ces valeurs de bio-impédances locales peuvent ensuite être additionnées pour déterminer une valeur de bio-impédance du tractus digestif correspondant à une mesure sur une étendue plus globale, et donc plus fiable.

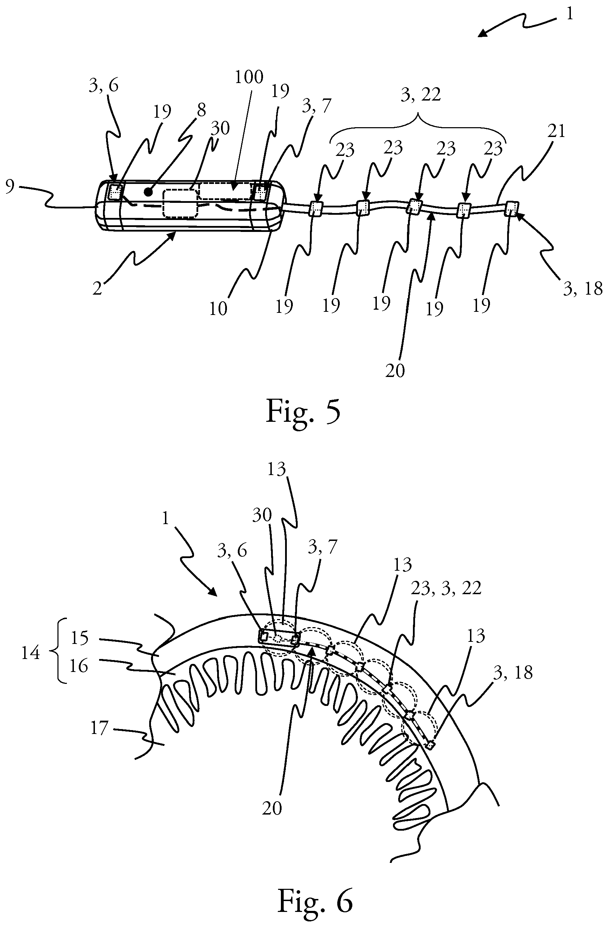

La figure 5 illustre un troisième mode de réalisation dans lequel le support d'électrodes déportées 20 sert de support, entre la deuxième extrémité longitudinale du boîtier et la troisième électrode 18 disposée à l'extrémité libre du support 20, une ou plusieurs électrodes intermédiaires 23. A l'exception de ce qui concerne la suite d'électrodes intermédiaires, la description du dispositif de mesure 1 selon le deuxième mode de réalisation tel que présenté en figure 3 s'applique *mutatis mutandis* à la description de la figure 5 et on pourra s'y reporter pour la mise en œuvre et la compréhension de la divulgation.

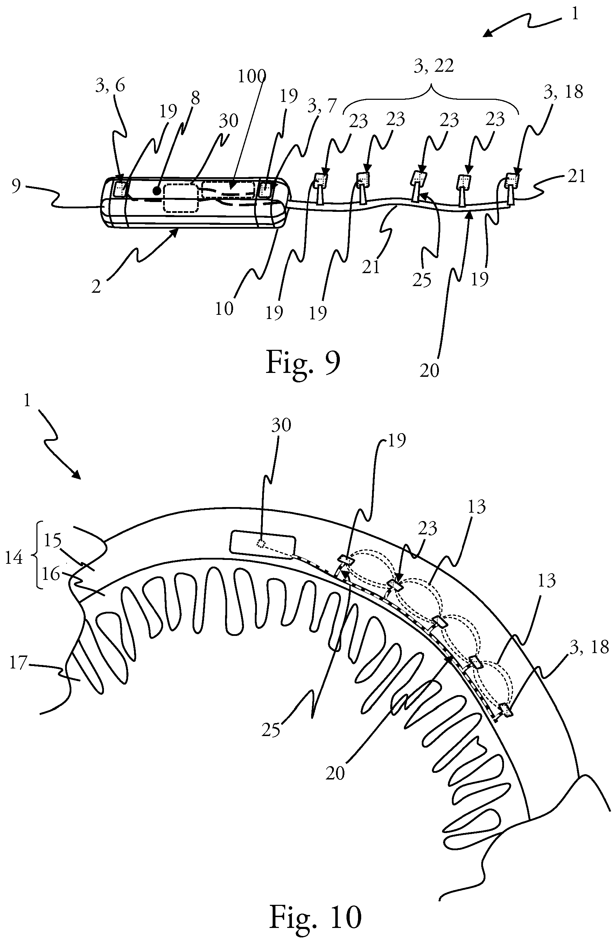

Dans ce mode de réalisation et dans ceux qui suivent, dans lesquels au moins deux électrodes sont déportées hors du boîtier, avec au moins la troisième électrode 18 disposée en extrémité du support 20 et au moins une électrode intermédiaire disposée sur ce support entre la troisième électrode et le boîtier, il pourra être prévu, tel que cela est représenté sur les figures 9 à 12 par exemple, que l'ensemble des électrodes soit déportée et que toutes les électrodes s'étendent ainsi hors du boîtier.

En l'espèce, la suite d'électrodes intermédiaires comprend quatre électrodes intermédiaires 23. Tout comme la troisième électrode 18, les électrodes intermédiaires 23 sont reliées au support d'électrodes déportées 20. Le support d'électrodes déportées 20 est un support direct des électrodes de la suite d'électrodes intermédiaires, tout comme pour la troisième électrode 18.

Chaque électrode intermédiaire 23 est ici configurée pour à la fois émettre un courant électrique et à la fois récupérer un signal électrique pour permettre la mesure d'un potentiel électrique. En cela, toutes les électrodes du jeu 3 d'électrodes, incluant les électrodes intermédiaires 23, peuvent être semblables.

Une distance égale sépare ici chaque électrode intermédiaire 23 de la série 22 d'électrodes intermédiaires. Pour l'ensemble du dispositif de mesure 1, chaque électrode du jeu 3 d'électrodes est séparée de l'électrode immédiatement adjacente par cette même distance.

Chaque électrode intermédiaire 23 est reliée électriquement, indépendamment des autres électrodes, au générateur électrique 30 et/ou au moyen de mesure de différence de potentiel électrique. Tel que décrit précédemment, ces raccordements électriques sont notamment réalisés par des cordons conducteurs indépendants les uns des autres et reliant respectivement une électrode au générateur.

La figure 6 présente le dispositif de mesure 1 illustré en figure 5, implanté dans le tissu sous-muqueux 15. Pour les autres éléments illustrés par la figure 6, l'on pourra se reporter *mutatis mutandis* à la description faite pour la figure 2. On pourra ainsi se référer à la figure 2 pour la mise en œuvre et la compréhension de l'invention selon le mode de réalisation décrit en figure 6.

Dans cet exemple de réalisation, la distance, ici égale, qui sépare les électrodes immédiatement adjacentes du jeu 3 d'électrodes permet la formation de boucles de circulation 13 locales, chacune fermé pour deux électrodes voisines du jeu 3 d'électrodes, et ce de proche en proche. Les électrodes du jeu 3 d'électrodes sont aptes à émettre dans plusieurs directions de sorte à ce que les boucles de circulation 13 locales traversent l'un ou l'autre des tissus voisins du jeu 3 d'électrodes.

Ainsi, dans le procédé de mesure de congestion du tractus digestif selon l'invention, la mesure de bio-impédance peut être déterminée via une mesure de différence de potentiel électrique global calculée en faisant la somme d'une pluralité de mesures locales de différence de potentiel électrique, dès lors que le courant injecté localement lors des mesures de différences de potentiels est le même d'une mesure locale à l'autre, chaque mesure locale de différence de potentiel électrique résultant d'une mesure faite aux bornes d'un couple d'électrodes du dispositif de mesure 1. Alternativement, il peut être prévu de déterminer des valeurs de bio-impédance locales, qui sont calculées respectivement à partir des mesures locales de différence de potentiel électrique. Ces valeurs de bio-impédances locales peuvent ensuite être additionnées pour déterminer une valeur de bio-impédance du tractus digestif correspondant à une mesure sur une étendue plus globale, et donc plus fiable.

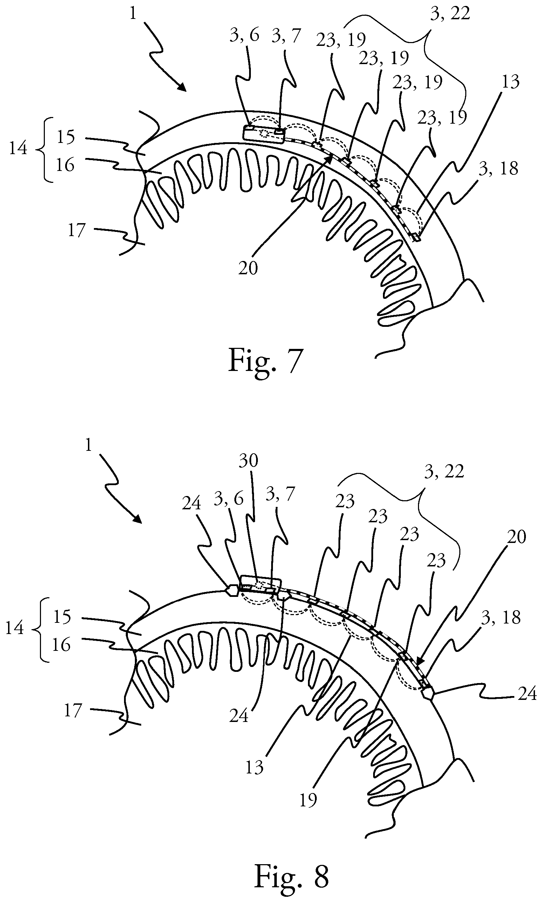

La figure 7 et la figure 8 et illustrent des modes de réalisation où la troisième électrode 18 et les électrodes intermédiaires 23 de la suite d'électrodes intermédiaires comprennent chacune leur surface d'interface 19 avec le tissu du tractus gastro-intestinal 14 de l'utilisateur tel que les surfaces d'interface 19 de la troisième électrode 18 et des électrodes intermédiaires 23 sont orientées dans une même direction et un même sens. La direction et le sens sont appréciés par rapport à la normale au plan d'extension de chaque surface d'interface 19, tel que précédemment décrit. De façon particulière, la première électrode 6 et la deuxième électrode 7 ont également leur surface d'interface 19 orientée dans cette même direction. Lors du positionnement du dispositif de mesure 1, on peut viser à ce que chaque surface d'interface 19 du jeu 3 d'électrodes soit positionnée de sorte à être dirigée au cœur du tissu du tractus gastro-intestinal 14, de façon spécifique. Cette configuration permet aux boucles de circulation 13 locales de parcourir dans leur intégralité le tissu du tractus gastro-intestinal 14, ici le tissu sous-muqueux 15. Dans le jeu 3 d'électrodes, chaque surface d'interface 19 peut être opposée à une surface isolante électriquement permettant d'orienter spécifiquement le courant injecté dans le tissu et d'éviter ainsi que la boucle de circulation 13 puisse se former du côté du dispositif opposé aux électrodes. Par exemple, la surface isolante électriquement est couverte de silicone.

Dans l'exemple de la figure 7, la suite d'électrodes intermédiaires 23 et la troisième l'électrode 18 sont implantées au sein du tissu sous-muqueux 15. Chaque surface d'interface 19 du jeu 3 d'électrodes est positionnée de sorte à être dirigée vers le cœur du tissu sous-muqueux 15, et non du côté de la muqueuse 16.

Dans l'exemple de la figure 8, chaque surface d'interface 19 du jeu 3 d'électrodes est disposée en surface du tissu gastrique 14 à l'opposé de la cavité 17 de l'estomac. On comprend que la représentation de la figure 8 est schématique et qu'une couche de tissu additionnel pourrait être disposé entre le tissu sous-muqueux 15 et les surfaces d'interface d'électrodes dès lors que conformément à ce qui vient d'être décrit, les électrodes sont disposées à l'opposé de la cavité 17 de l'estomac par rapport au tissu du tractus digestif. En d'autres termes, le jeu 3 d'électrodes pourrait être disposé au contact de tout autre tissu gastrique 14 sans sortir du cadre de l'invention, dès lors que chaque surface d'interface 19 du jeu 3 d'électrodes est positionnée de sorte à être dirigée vers le cœur dudit tissu gastrique 14.

Pour permettre cette configuration décrite en figure 8 et pour maintenir le jeu 3 d'électrodes en position, le dispositif de mesure 1 comprend au moins un dispositif de fixation 24 au tissu du tractus gastro-intestinal 14. Ici chaque extrémité du boîtier 2, à savoir la première extrémité longitudinale 9 et la deuxième extrémité longitudinale 10, comprend un dispositif de fixation 24 au tissu 14, ce qui permet de maintenir la position de la première électrode 6 et de la deuxième électrode 7. Un ou plusieurs dispositifs de fixation peuvent être répartis sur le support d'électrodes déportées 20. Avantageusement, un dispositif de fixation 24 est situé au moins au voisinage de la troisième électrode 18, de sorte à maintenir la troisième électrode 18 qui est l'électrode la plus déportée par rapport au boîtier 2.

On comprend que l'intérêt de tel dispositifs de fixation est tout aussi fort pour les dispositifs des autres modes de réalisation, et qu'il peut être prévu selon la divulgation d'équiper les boîtiers ou les cordons conducteurs d'électricité de tel dispositif de fixation.

La figure 9 illustre un mode de réalisation de la divulgation, dans lequel la troisième électrode 18 et les électrodes intermédiaires 23 sont décalées latéralement par rapport à la direction d'allongement du support d'électrodes déportées 20. La troisième électrode 18 et les électrodes intermédiaires 23 sont chacune reliées au support d'électrodes déportées 20 par une branche 25 conductrice de courant. La gaine périphérique 21 isolante est configurée pour couvrir également chacune des branches 25 conductrices de courant, ou bien des gaines isolantes propres à chaque branche sont raccordées à la gaine périphérique 21 isolante. Le courant est ainsi apte à circuler depuis le générateur de courant 30, via le support d'électrodes déportées 20 vers chacune des électrodes décalées latéralement, de telle sorte que cette position n'empêche pas le raccordement au générateur électrique, indépendamment les unes des autres, de l'ensemble des électrodes.

Tel que cela a été évoqué précédemment, ce mode de réalisation est également particulier en ce que la première électrode et la deuxième électrode précédemment présentes dans le boîtier ont ici été retirées. De la sorte, l'ensemble des électrodes est déporté et toutes les électrodes respectivement alimentées par le générateur sont disposées à distance du boîtier. On comprendra que les modes de réalisation précédemment décrits dans lesquels au moins deux électrodes s'étendent en dehors du boîtier pourraient également être aménagés de la sorte, avec aucune électrode intégrée au boîtier.

La figure 10 montre le dispositif de mesure 1 de congestion du tractus digestif en situation, implanté dans le tissu du tractus gastro-intestinal 14 de l'utilisateur. Chaque électrode du jeu 3 d'électrodes génère, comme présenté en figure 8, des boucles de circulation 13 locales au sein du tissu sous-muqueux 15. Les boucles de circulation 13 locales sont décalées latéralement par rapport à ce qui a été présenté en figure 8, de sorte à être éloignées du support d'électrodes déportées 20 et, de ce fait, de la muqueuse 16 situé au voisinage de ce support d'électrodes déportées 20. Les boucles de circulation 13 locales traversent alors un tissu sain ou moins impacté par une dégénérescence fibreuse pouvant résulter de l'implantation du support d'électrodes déportées 20. Ainsi, les mesures de différence de potentiel électrique locales sont indépendantes d'artefacts pouvant survenir avec la dégénérescence fibreuse du tissu du tractus gastro-intestinal 14.

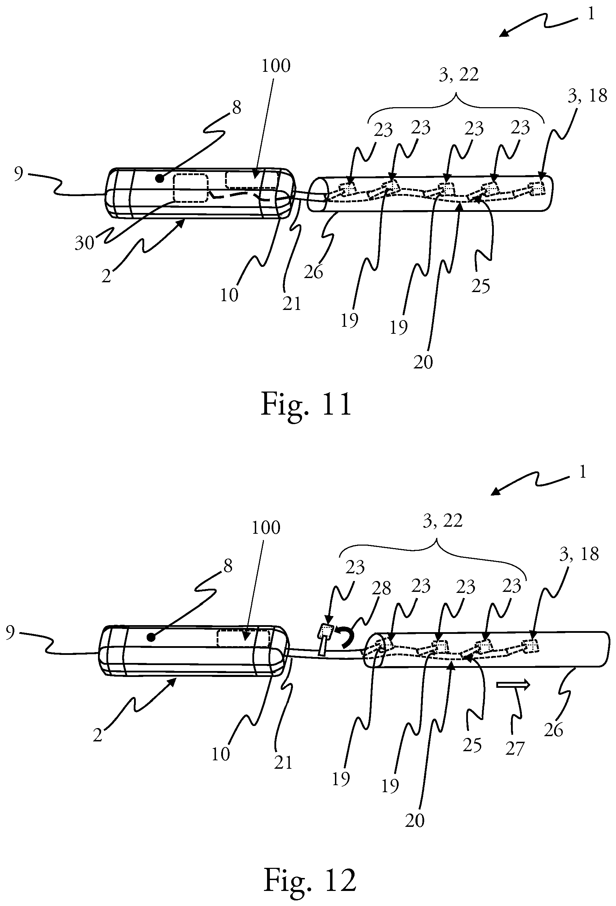

La figure 11 et la figure 12 illustrent le dispositif de mesure 1 dans lequel la branche 25 conductrice de courant est déployable dans le tissu du tractus gastro-intestinal 14. La description faite à la figure 9 s'applique *mutatis mutandis* à la description des figures 11 et 12 et l'on pourra se reporter à la description de la figure 9 pour mettre en œuvre et comprendre la divulgation.

Les figures 11 et 12 présentent un exemple de mise en œuvre de la déployabilité de la branche 25 conductrice de courant. La branche 25 conductrice de courant déployable voit son utilité par exemple lors de l'implantation du support d'électrodes déportées 20 au sein du tissu du tractus gastro-intestinal 14. Alors, la déployabilité de la branche 25 conductrice de courant permet d'éloigner les électrodes du jeu 3 d'électrodes sans déchirer le tissu gastrique et donc avec un impact plus faible sur le tissu du tractus gastro-intestinal 14. La branche 25 conductrice de courant déployable peut également être utile pour orienter les électrodes dans le tissu du tractus gastro-intestinal 14 ou à la surface externe 8 de celui-ci.

De la sorte, on évite l'inconvénient d'implanter un dispositif avec des branches déjà déployées ce qui aurait pour effet de déchirer des fibres à l'insertion et de créer à nouveau une reformation de fibres autour des électrodes.

Lors de l'installation du dispositif de mesure 1 de congestion du tractus digestif dans l'utilisateur, les branches 25 conductrices de courant sont susceptibles de s'accrocher dans les tissus lors de l'installation du dispositif de mesure 1. Il est ainsi possible de recouvrir le support d'électrodes déportées 20 d'un étui de protection 26 comme présenté en figure 11. Cet étui de protection 26 sera enlevé une fois le dispositif de mesure 1 positionné. L'étui de protection 26 est enlevé comme présenté en figure 12.

Comme montré en figure 11, lorsque le support d'électrodes déportées 20 est couvert de l'étui de protection 26, les branches 25 conductrices de courant sont préférentiellement en position repliée ou rétractée le long du support d'électrodes déportées 20 pour occuper un volume moindre et éviter qu'elles ne soient détériorées.

Comme montré en figure 12, lorsque le support d'électrodes déportées 20 est découvert et l'étui de protection 26 enlevé, les branches 25 conductrices de courant passent en position déployée comme l'illustre la flèche pleine 28. Pour ce faire, un mécanisme de déploiement, ici non représenté, est actionné par exemple automatiquement lorsque l'étui de protection 26 ne contraint plus le déploiement de la branche 25 conductrice de courant.

L'étui de protection 26 est enlevé une fois le support d'électrodes déportées 20 positionné pour sa mise en œuvre. De préférence, l'étui de protection 26 est enlevé dans un sens inverse au sens d'insertion du boîtier 2 comme illustré par une flèche 27, empruntant ainsi un passage ménagé dans l'utilisateur à l'insertion du boîtier 2.

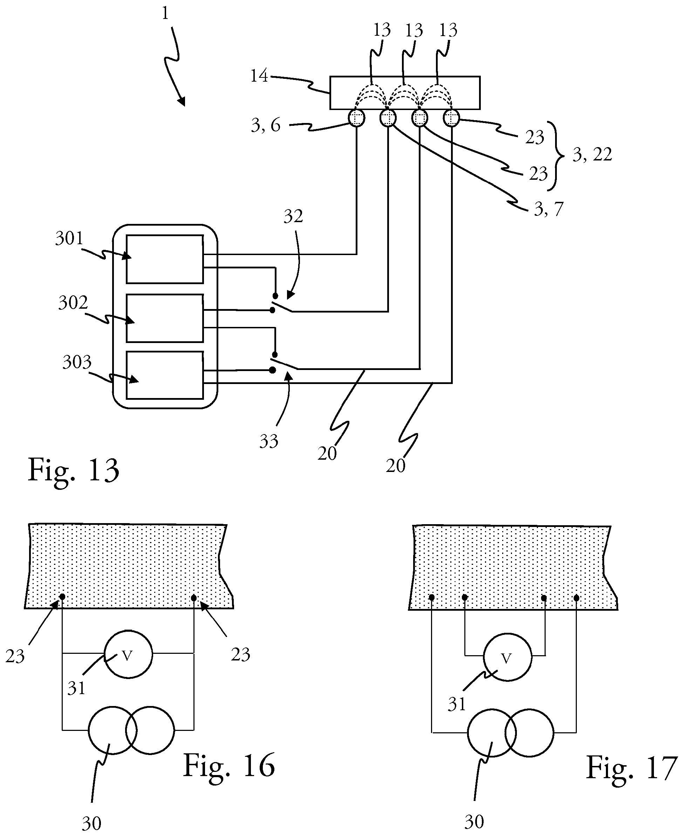

La figure 13, la figure 14 et la figure 15 illustrent des schémas de raccordement électrique appropriés pour le bon fonctionnement du dispositif de mesure selon la divulgation.

La figure 13 illustre une unité de contrôle comprenant une pluralité de sous-modules 300, 301, 302 formant respectivement une puce de commande intégrant le générateur de courant et le moyen de mesure de différence de potentiel électrique. Chaque sous-module 300, 301, 302 est relié électriquement à deux électrodes, avec au moins une des électrodes qu'il relie qui est également reliée à un autre sous-module 300, 301, 302. A titre d'exemple, un premier sous-module 301 est relié à la première électrode 6 et à la deuxième électrode 7 et un deuxième sous-module 302 est relié à cette même deuxième électrode 7 et une électrode intermédiaire 23. Lorsque du courant est généré au niveau du premier sous-module 301, un commutateur 32 assure que la deuxième électrode 7 soit reliée à ce premier sous-module 301 et la mesure de différence de potentiel se fait au niveau du premier sous-module 301 entre la première électrode 6 et la deuxième électrode 7. Lorsqu'ensuite du courant est généré au niveau du deuxième sous-module 302, un commutateur 33 assure que la deuxième électrode 7 soit reliée cette fois à ce deuxième sous-module 302 et que l'électrode voisine, à savoir ici l'électrode intermédiaire 23, soit également reliée au deuxième sous-module 302 : la mesure de différence de potentiel se fait alors au niveau du deuxième sous-module 302 entre la deuxième électrode 7 et l'électrode intermédiaire 23.

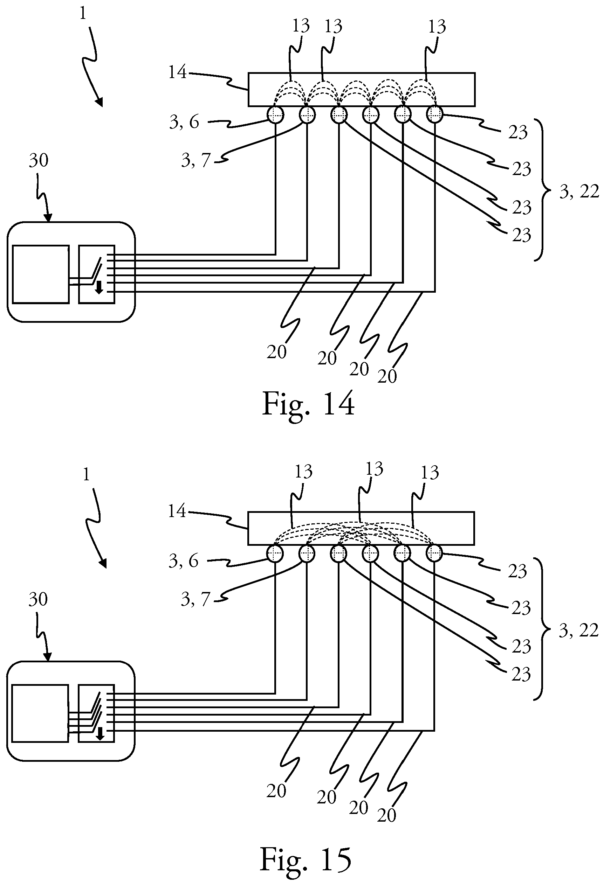

La figure 14 et la figure 15 illustrent des variantes de mesure à deux ou quatre électrodes, notamment dans le cadre d'une pluralité de mesures locales permettant d'obtenir une mesure globale de différence de potentiel.

Sur la figure 14, on a par exemple illustré un pilotage des commutateurs au niveau du générateur de courant 30 qui permet de former des boucles de circulation de proche en proche incluant deux électrodes immédiatement voisines. On parle ici de mesures locales à deux électrodes. Plus particulièrement, le raccordement de ces électrodes est illustré sur la figure 16. Pour une suite comportant N électrodes 23, 18, depuis le boîtier jusqu'à l'extrémité libre du support d'électrode sur lequel est disposée la troisième électrode, on comprend que lorsqu'un commutateur est commandé pour alimenter en courant une électrode n, un commutateur est alors piloté pour relier électriquement l'électrode n+1 directement voisine au même générateur de courant de manière à ce que le voltmètre associé récupère une information de différence de potentiel électrique, puis ce même commutateur est piloté pour relier cette fois l'électrode n+1 et l'électrode n+2 directement voisines au même générateur de courant de manière à ce que le voltmètre associé récupère une autre information de différence de potentiel électrique, et ainsi de suite, de proche en proche, pour récupérer l'ensemble des informations de différence de potentiel électrique.

Sur la figure 15, on a illustré une variante du dispositif selon l'invention dans laquelle est mise en œuvre une mesure locale à quatre électrodes, avec des ensembles de quatre électrodes qui peuvent être formés de proche en proche par le pilotage approprié des commutateurs. Plus particulièrement, le raccordement de ces ensembles d'électrodes est illustré sur la figure 17. Les commutateurs sont pilotés de sorte que les électrodes soient alimentées quatre à quatre, par ensembles de deux électrodes. La mesure globale se fait là encore par la prise en compte de plusieurs mesures locales.

Tel qu'évoqué précédemment, la figure 16 illustre un montage permettant une mesure à deux électrodes, le moyen de mesure de différence de potentiel 31 étant raccordé aux bornes du générateur de tension 30 auquel sont connectées respectivement chacune des électrodes 23, noyées ici dans le tissu sous-muqueux 15. Et la figure 17 illustre un montage différent de celui illustré par la figure 16, en ce qu'il permet une mesure à quatre électrodes, avec une paire d'électrodes reliée au générateur pour la formation de la boucle de circulation dans le tissu sous-muqueux 15, et une paire d'électrodes reliée directement au moyen de mesure de différence de potentiel 31. Une mesure à quatre électrodes permet de minimiser l'impédance de contact due aux électrodes.

Les différents modes de réalisation précédemment décrits et illustrés peuvent être mis en œuvre avec l'un ou l'autre de ces montages.

On comprend à la lecture de ce qui précède que la présente divulgation propose un dispositif de mesure de congestion du tractus digestif configuré pour fiabiliser la détection d'un œdème pulmonaire révélateur d'une décompensation cardiaque. Ce dispositif de mesure, destiné notamment à être implanté dans ou contre les tissus gastriques d'un utilisateur, facilite le suivi régulier des utilisateurs à risque. L'efficacité de la mesure de bio-impédance du tractus digestif est augmentée dans les différents modes de réalisation de la divulgation, grâce à la spécificité tissulaire et la stabilité de positionnement des électrodes mises en œuvre. Par ailleurs, la combinaison de cette mesure de bio-impédance avec une mesure additionnelle distincte, mécanique, apte à mettre en évidence des indications de modifications de la morphologie ou de la structure de la paroi gastrique, permet de fiabiliser l'analyse relative à la teneur en eau de la paroi gastrique en quantité trop importante.

## Revendications

1. Dispositif de mesure (1) de congestion du tractus digestif d'un utilisateur, comprenant au moins un boîtier (2), un générateur de courant (30) et un moyen de mesure de différence de potentiel (31) logés dans ledit boîtier, et un jeu d'électrodes (3) comportant au moins deux électrodes raccordées électriquement, indépendamment l'une de l'autre, au générateur de courant (30) et/ou au moyen de mesure de différence de potentiel (31) aux bornes des électrodes, chaque électrode du jeu (3) d'électrodes étant configurée pour émettre un courant électrique et/ou permettre la mesure d'une différence de potentiel électrique, le jeu (3) d'électrodes est configuré pour générer au moins une boucle de circulation (13) du courant électrique circulant au moins au travers d'un tissu du tractus gastro-intestinal (14) de l'utilisateur et pour permettre la mesure d'une différence de potentiel électrique relatif au tissu du tractus gastro-intestinal (14), le dispositif de mesure (1) comprenant en outre un module de calcul (5) configuré pour recevoir la différence de potentiel électrique mesurée et pour calculer une valeur de bio-impédance du tractus digestif en fonction de cette mesure relative au tissu (14) et le dispositif de mesure comportant des moyens (100) permettant une mesure mécanique d'une caractéristique structurelle ou morphologique de la paroi gastrique dans la même zone que celle sur laquelle a été effectuée la mesure de bio-impédance et simultanément, les moyens permettant une mesure mécanique d'une caractéristique structurelle ou morphologique de la paroi gastrique comportant un accéléromètre (100), le module de calcul (5) étant apte à recevoir la différence de potentiel électrique mesurée entre les deux électrodes du jeu (3) d'électrodes, de manière à calculer la valeur de bio-impédance du tractus digestif, et des valeurs de signaux mesurés par l'accéléromètre (100), pour calculer une valeur représentative d'une caractéristique structurelle ou morphologique du tractus digestif, dans la même zone que celle sur laquelle a été effectuée la mesure de bio-impédance,
le dispositif de mesure (1) est **caractérisé en ce que** le module de calcul (5) est configuré pour comparer la valeur représentative de la caractéristique structurelle ou morphologique du tractus digestif et la valeur mesurée de bio-impédance avec une valeur seuil qui leur est associée et qui est stockée dans une mémoire du module de calcul (5).

2. Dispositif de mesure selon la revendication 1, **caractérisé en ce que** les moyens (100) permettant une mesure mécanique d'une caractéristique structurelle ou morphologique de la paroi gastrique sont intégrés dans le boîtier (2).

3. Dispositif de mesure (1) selon l'une des revendications précédentes, dans lequel le jeu (3) d'électrodes comporte deux électrodes parmi lesquelles une première électrode (6) disposée à une première extrémité longitudinale (9) du boîtier (2) et une deuxième électrode (7) disposée à une deuxième extrémité longitudinale (10) opposée du boîtier (2).

4. Dispositif de mesure (1) selon l'une quelconque des revendications précédentes, dans lequel le jeu (3) d'électrodes comprend une pluralité d'électrodes parmi lesquelles au moins une électrode (18, 23) est déportée à l'extérieur du boîtier (2), et dans lequel un support d'électrodes déportées (20) externe au boîtier (2) est configurée pour relier le générateur de courant (30) et/ou le moyen de mesure de différence de potentiel (31) à ladite électrode déportée (18, 23), le support d'électrodes déportées (20) comprenant une gaine périphérique (21) isolante s'étendant entre le boîtier (2) et ladite électrode déportée (18).

5. Dispositif de mesure (1) selon la revendication précédente, dans lequel toutes les électrodes du jeu d'électrodes (3) sont déportées à l'extérieur du boîtier, la gaine périphérique isolante (21) logeant, de manière isolée électriquement les uns par rapport aux autres, une pluralité de cordons conducteurs d'électricité configurés pour relier les électrodes déportées au générateur de courant (30) et/ou au moyen de mesure de différence de potentiel (31), indépendamment les unes des autres.

6. Dispositif de mesure (1) selon la revendication 4 ou 5, dans lequel le jeu d'électrodes comporte au moins une électrode intermédiaire (23) s'étendant à l'extérieur du boîtier entre le boîtier (2) et ladite électrode déportée (18) formant l'électrode la plus éloignée du boîtier, l'au moins une électrode intermédiaire (23) étant disposée à l'extrémité d'une branche (25) conductrice de courant et isolée du tissu du tractus gastro-intestinal par une gaine isolante, ladite branche (25) étant déployable par rapport au support (20) muni de la gaine périphérique isolante (21).

7. Dispositif de mesure (1) selon la revendication 4 ou 5, dans lequel le jeu d'électrodes comporte une pluralité d'électrodes reliées électriquement au générateur de courant (30) et/ou au moyen de mesure de différence de potentiel (31), le dispositif de mesure comportant un jeu de commutateurs (32) disposés sur les liaisons électriques indépendantes les unes des autres de chaque électrode avec le générateur de courant (30) et/ou le moyen de mesure de différence de potentiel (31), le module de calcul (5) étant configuré pour piloter le commutateur pour déterminer quelles électrodes du jeu sont mises en œuvre pour émettre et mesurer le courant traversant le tissu du tractus gastro-intestinal.

8. Dispositif de mesure (1) selon l'une des revendications précédentes, dans lequel chaque électrode est reliée à la fois au générateur de courant (30) pour réaliser l'émission dans le tissu du tractus gastro-intestinal d'un courant électrique et au moyen de mesure de différence de potentiel (31).

9. Dispositif de mesure (1) selon l'une des revendications 1 à 8, dans lequel au moins une paire d'électrodes est reliée aux bornes du générateur de courant (30) pour participer à l'émission d'une boucle de circulation de courant dans le tissu du tractus gastro-intestinal, et au moins une paire d'électrodes est reliée aux bornes du moyen de mesure de différence de potentiel (31).

10. Dispositif de mesure (1) selon l'une quelconque des revendications précédentes, comprenant au moins un dispositif de fixation (24) au tissu du tractus gastro-intestinal (14), disposé à au moins une extrémité du boîtier (2) et/ ou sur le support d'électrodes déportées (20) et/ou sur la branche (25) conductrice de courant.

## Patentansprüche

1. Vorrichtung (1) zur Messung von Verstopfungen im Verdauungstrakt eines Benutzers, bestehend aus mindestens einem Gehäuse (2), einem Stromgenerator (30) und einer darin untergebrachten Potentialdifferenzmesseinrichtung (31) sowie einem Elektrodensatz (3), der mindestens zwei Elektroden umfasst, die unabhängig voneinander mit dem Stromgenerator (30) und/oder der Potentialdifferenzmesseinrichtung (31) an den Elektrodenanschlüssen elektrisch verbunden sind, wobei jede Elektrode des Elektrodensatzes (3) ist so konfiguriert, dass sie elektrischen Strom abgibt und/oder die Messung einer elektrischen Potentialdifferenz ermöglicht, wobei der Elektrodensatz (3) ist so konfiguriert, dass er mindestens einen Kreislauf (13) des elektrischen Stroms erzeugt, der zumindest durch ein Gewebe des Magen-Darm-Trakts (14) des Benutzers fließt, und die Messung einer elektrischen Potentialdifferenz relativ zum Gewebe des Magen-Darm-Trakts (14) ermöglicht, wobei die Messvorrichtung (1) umfasst ferner ein Berechnungsmodul (5), das die gemessene elektrische Potentialdifferenz empfängt und einen Bioimpedanzwert des Verdauungstrakts basierend auf dieser Messung des Gewebes (14) berechnet, wobei das Messgerät umfasst Mittel (100) zur mechanischen Messung einer strukturellen oder morphologischen Eigenschaft der Magenwand im Bereich der Bioimpedanzmessung, wobei die Mittel zur mechanischen Messung einer strukturellen oder morphologischen Eigenschaft der Magenwand umfassen einen Beschleunigungsmesser (100), wobei das Berechnungsmodul (5) empfängt die zwischen den beiden Elektroden des Elektrodensatzes (3) gemessene elektrische Potenzialdifferenz zur Berechnung des Bioimpedanzwerts des Verdauungstrakts und berechnet aus den vom Beschleunigungsmesser (100) gemessenen Signalwerten einen Wert, der eine strukturelle oder morphologische Eigenschaft des Verdauungstrakts im Bereich der Bioimpedanzmessung repräsentiert, wobei das Messgerät ist **dadurch gekennzeichnet, dass** das Berechnungsmodul (5) den Wert, der die strukturelle oder morphologische Eigenschaft des Verdauungstrakts repräsentiert, und den gemessenen Bioimpedanzwert mit einem zugehörigen, in einem Speicher abgelegten Schwellenwert vergleicht des Berechnungsmoduls (5).

2. Messvorrichtung (1) nach Anspruch 1, **dadurch gekennzeichnet, dass** die Mittel (100) zur mechanischen Messung einer strukturellen oder morphologischen Eigenschaft der Magenwand in das Gehäuse (2) integriert sind.

3. Messvorrichtung (1) nach einem der vorhergehenden Ansprüche, wobei der Elektrodensatz (3) zwei Elektroden umfasst, darunter eine erste Elektrode (6), die an einem ersten Längsende (9) des Gehäuses (2) angeordnet ist, und eine zweite Elektrode (7), die an einem dem Gehäuse (2) gegenüberliegenden zweiten Längsende (10) angeordnet ist.

4. Messvorrichtung (1) nach einem der vorhergehenden Ansprüche, wobei der Elektrodensatz (3) mehrere Elektroden umfasst, von denen mindestens eine Elektrode (18, 23) außerhalb des Gehäuses (2) versetzt ist, und wobei ein Träger für versetzte Elektroden (20) außerhalb des Gehäuses (2) so konfiguriert ist, dass er den Stromgenerator (30) und/oder die Einrichtung (31) zur Messung der Potentialdifferenz mit der versetzten Elektrode (18, 23) verbindet, wobei der Träger für versetzte Elektroden (20) eine isolierende periphere Hülle (21) umfasst, die sich zwischen dem Gehäuse (2) und der versetzten Elektrode (18) erstreckt.

5. Messvorrichtung (1) nach dem vorhergehenden Anspruch, wobei alle Elektroden des Elektrodensatzes (3) außerhalb des Gehäuses versetzt sind, wobei die isolierende periphere Hülle (21) elektrisch voneinander isoliert mehrere elektrisch leitfähige Kabel aufnimmt, die so konfiguriert sind, dass sie die versetzten Elektroden unabhängig voneinander mit dem Stromgenerator (30) und/oder der Einrichtung (31) zur Messung der Potentialdifferenz verbinden.

6. Messvorrichtung (1) nach Anspruch 4 oder 5, wobei der Elektrodensatz mindestens eine Zwischenelektrode (23) umfasst, die sich außerhalb des Gehäuses zwischen dem Gehäuse (2) und der entfernten Elektrode (18) erstreckt, die die am weitesten vom Gehäuse entfernte Elektrode bildet, wobei die mindestens eine Zwischenelektrode (23) am Ende eines stromführenden Zweigs (25) angeordnet und durch eine isolierende Hülle vom Gewebe des Magen-Darm-Trakts isoliert ist, wobei der Zweig (25) relativ zu dem mit der isolierenden peripheren Hülle (21) versehenen Träger (20) ausfahrbar ist.

7. Messvorrichtung (1) nach Anspruch 4 oder 5, wobei der Elektrodensatz mehrere Elektroden umfasst, die elektrisch mit dem Stromgenerator (30) und/oder der Potentialdifferenzmesseinrichtung (31) verbunden sind, wobei das Messgerät umfasst einen Satz Schalter (32), die an den voneinander unabhängigen elektrischen Verbindungen jeder Elektrode mit dem Stromgenerator (30) und/oder der Potentialdifferenzmesseinrichtung (31) angeordnet sind, wobei das Berechnungsmodul (5) ist so konfiguriert, dass es die Schalter steuert, um zu bestimmen, welche Elektroden des Satzes zur Abgabe und Messung des durch das Gewebe des Magen-Darm-Trakts fließenden Stroms verwendet werden.

8. Messvorrichtung (1) nach einem der vorhergehenden Ansprüche, wobei jede Elektrode sowohl mit dem Stromgenerator (30) zur Abgabe eines elektrischen Stroms in das Gewebe des Magen-Darm-Trakts als auch mit der Potentialdifferenzmesseinrichtung (31) verbunden ist.

9. Messvorrichtung (1) nach einem der Ansprüche 1 bis 8, wobei mindestens ein Elektrodenpaar mit den Anschlüssen des Stromgenerators (30) verbunden ist, um an der Emission einer Stromzirkulationsschleife im Gewebe des Magen-Darm-Trakts teilzunehmen, und mindestens ein Elektrodenpaar mit den Anschlüssen der Potentialdifferenzmesseinrichtung (31) verbunden ist.

10. Messvorrichtung (1) nach einem der vorhergehenden Ansprüche, umfassend mindestens eine Vorrichtung (24) zur Befestigung am Gewebe des Magen-Darm-Trakts (14), die an mindestens einem Ende des Gehäuses (2) und/oder am entfernten Elektrodenträger (20) und/oder am stromführenden Zweig (25) angeordnet ist.

## Claims

1. Device (1) for measuring congestion of the digestive tract of a user, comprising at least one housing (2), a current generator (30) and a potential difference measuring means (31) housed in said housing, and a set of electrodes (3) comprising at least two electrodes electrically connected, independently of one another, to the current generator (30) and/or to the potential difference measuring means (31) at the terminals of the electrodes, each electrode of the set (3) of electrodes being configured to emit an electric current and/or to allow the measurement of an electric potential difference, the set (3) of electrodes is configured to generate at least one circulation loop (13) of the electric current circulating at least through a tissue of the gastrointestinal tract (14) of the user and to allow the measurement of an electric potential difference relative to the tissue of the gastrointestinal tract (14), the measuring device (1) further comprising a calculation module (5) configured to receive the measured electrical potential difference and to calculate a bio-impedance value of the digestive tract as a function of this measurement relating to the tissue (14) and the measuring device comprising means (100) allowing a mechanical measurement of a structural or morphological characteristic of the gastric wall in the same area as that on which the bio-impedance measurement was carried out and simultaneously, the means allowing a mechanical measurement of a structural or morphological characteristic of the gastric wall comprising an accelerometer (100), the calculation module (5) being able to receive the electrical potential difference measured between the two electrodes of the set (3) of electrodes, so as to calculate the bio-impedance value of the digestive tract, and values of signals measured by the accelerometer (100), to calculate a value representative of a structural or morphological characteristic of the digestive tract, in the same area as that on which the bio-impedance measurement was carried out,
the measuring device is **characterized in that** the calculation module (5) is configured to compare the value representative of the structural or morphological characteristic of the digestive tract and the measured bioimpedance value with a threshold value associated therewith and stored in a memory of the calculation module (5).

2. Measuring device according to claim 1, **characterized in that** the means (100) allowing a mechanical measurement of a structural or morphological characteristic of the gastric wall are integrated in the housing (2).

3. Measuring device (1) according to one of the preceding claims, in which the set (3) of electrodes comprises two electrodes, including a first electrode (6) arranged at a first longitudinal end (9) of the housing (2) and a second electrode (7) arranged at a second longitudinal end (10) opposite the housing (2).

4. Measuring device (1) according to any one of the preceding claims, wherein the set (3) of electrodes comprises a plurality of electrodes among which at least one electrode (18, 23) is offset outside the housing (2), and wherein a support for offset electrodes (20) external to the housing (2) is configured to connect the current generator (30) and/or the potential difference measuring means (31) to said offset electrode (18, 23), the support for offset electrodes (20) comprising an insulating peripheral sheath (21) extending between the housing (2) and said offset electrode (18).

5. Measuring device (1) according to the preceding claim, in which all the electrodes of the set of electrodes (3) are offset outside the housing, the insulating peripheral sheath (21) housing, in an electrically insulated manner from one another, a plurality of electrically conductive cords configured to connect the offset electrodes to the current generator (30) and/or to the potential difference measuring means (31), independently of one another.

6. Measuring device (1) according to claim 4 or 5, wherein the set of electrodes comprises at least one intermediate electrode (23) extending outside the housing between the housing (2) and said remote electrode (18) forming the electrode furthest from the housing, the at least one intermediate electrode (23) being arranged at the end of a current-conducting branch (25) and insulated from the tissue of the gastrointestinal tract by an insulating sheath, said branch (25) being deployable relative to the support (20) provided with the insulating peripheral sheath (21).

7. Measuring device (1) according to claim 4 or 5, wherein the set of electrodes comprises a plurality of electrodes electrically connected to the current generator (30) and/or to the potential difference measuring means (31), the measuring device comprising a set of switches (32) arranged on the mutually independent electrical connections of each electrode with the current generator (30) and/or the potential difference measuring means (31), the calculation module (5) being configured to control the switch to determine which electrodes of the set are used to emit and measure the current passing through the tissue of the gastrointestinal tract.

8. Measuring device (1) according to one of the preceding claims, wherein each electrode is connected both to the current generator (30) to carry out the emission into the tissue of the gastrointestinal tract of an electric current and to the potential difference measuring means (31).

9. Measuring device (1) according to one of claims 1 to 8, wherein at least one pair of electrodes is connected to the terminals of the current generator (30) to participate in the emission of a current circulation loop in the tissue of the gastrointestinal tract, and at least one pair of electrodes is connected to the terminals of the potential difference measuring means (31).

10. Measuring device (1) according to any one of the preceding claims, comprising at least one device (24) for fixing to the tissue of the gastrointestinal tract (14), arranged at at least one end of the housing (2) and/or on the remote electrode support (20) and/or on the current-conducting branch (25).
